# EUROPEAN PATENT SPECIFICATION

(11) **EP 1 105 731 B1**
(45) Date of publication and mention of the grant of the patent: **18.10.2006**
(21) Application number: 99938998.4
(22) Date of filing: 04.08.1999
(51) Int. Cl.: G01N 33/53, G06F 17/30

(54) **PROFILING AND CATALOGING EXPRESSED PROTEIN TAGS**
DARSTELLUNG DES PROFILS UND KATALOGISIERUNG VON EXPRIMIERTEN PROTEINMARKERN
ETABLISSEMENT DE PROFIL ET CATALOGAGE DE MARQUEURS DE PROTEINES EXPRIMEES

(30) Priority: 12.08.1998 US 133094; 12.08.1998 US 96291 P; 25.05.1999 US 135728 P
(43) Date of publication of application: 13.06.2001
(73) Proprietor: Zycos Inc., Cambridge, MA 02138 (US)
(72) Inventor: CHICZ, Roman, M., Belmont, MA 02478 (US); HEDLEY, Mary, Lynne, Lexington, MA 02173 (US); HSU, Charles, San Francisco, CA 94115 (US); URBAN, Robert, G., Lexington, MA 02173 (US)
(74) Representative: Arends, William Gerrit
(86) International application number: PCT/US1999/017680
(87) International publication number: WO 2000/009654

(56) References cited:
- US-A- 5 692 181
- US-A- 5 741 653
- US-A- 5 778 363
- KASSENBROCK & R B KELLY C K: "Interaction of heavy chain binding protein (BiP/GRP78) with adenine nucleotides" EMBO JOURNAL, OXFORD UNIVERSITY PRESS, SURREY, GB, vol. 8, no. 5, 1989, pages 1461-1467, XP002130142 ISSN: 0261-4189
- PETROVECKI MLADEN ET AL: "Analysis of data on leukocyte surface markers for recognition of leukemia/lymphoma phenotype pattern." CLINICAL BIOCHEMISTRY, vol. 29, no. 1, 1996, pages 21-25, XP002205434 ISSN: 0009-9120
- CELIS J E ET AL.: "The human keratinocyte two-dimensional gel protein database (update 1995): Mapping components of signal transduction pathways." ELECTROPHORESIS, vol. 16, no. 12, 1995, pages 2177-2240, XP001095051

## Description

### Field of the Invention

The invention relates to the characterization of a cell's protein repertoire and the storage and manipulation of that information in a computer database.

### Background of the Invention

Essentially every cell within an organism contains the complete and identical genetic information of that organism, but each cell expresses only the small subset of genes specifically required for that given type of cell. For example, the human genome, which is composed of a total of three billion nucleotides, is thought to include ~100,000 genes. However, each individual cell expresses only about 2,000 to about 4,000 different proteins, corresponding to only ~2% to about 4% of the total number of genes. It is the concerted activity of the proteins expressed in a given cell which orchestrates all the required activities that define each particular cell type at a given developmental, metabolic or disease stage.

In the past decades it has become clear that the development and the pathology of many diseases involve differences in gene expression. Indeed, healthy and diseased tissue or cell types can frequently be distinguished by differences in gene expression. For example, normal cells may evolve to highly invasive and metastatic cancer cells by activation of certain growth-inducing genes, *e.g.,* oncogenes, or the inactivation of certain growth-inhibitory genes, e.g., tumor suppressors or apoptosis activators. Levine, 1997, *Cell* 88:323-331; Hunter, 1997, *Cell* 88:333-346; Jacobson, 1997, *Cell* 88:347-354; Nagata, 1997, *Cell* 88:355-365; Fraser *et al.,* 1996, *Cell* 85:781-784. Altered expression of such genes, e.g., growth activators or growth suppressors, in turn affects expression of other genes. See, The National Cancer Institute, "The Nation's Investment In Cancer Research: A Budget Proposal For Fiscal Years 1997/98", Prepared by the Director, National Cancer Institute, pp. 55-77.

Pathological gene expression differences are not confined to cancer. Autoimmune disorders, many neurodegenerative diseases, inflammatory diseases, restenosis, atherosclerosis, many metabolic diseases, and numerous others are believed to involve aberrant expression of particular genes. Naparstek *et al*., 1993, *Ann. Rev. Immunol.* 11:79-104; Sercarz *et al.,* 1993, *Ann. Rev. Immunol.* 11:729-766. As a consequence, the present day challenge in medical research is to understand the role each gene or its encoded protein plays in maintaining normal cellular homeostasis and to utilize this heightened understanding in improving our ability to treat disease and/or identify predispositions to disease at stages when more promising treatment or prevention methods are available. In particular, an efficient method allowing the assessment of the proteins expressed in a given cell, tissue or organ type, and the retrieval of the genetic information encoding differentially expressed proteins, would be an extremely valuable tool for genetic and medical research.

Significant resources have been expended in recent years to identify and isolate genes relevant to disease development. One approach which has been taken is to catalogue all the individual genes encoded by the chromosomes of a species. In the case of humans, the NIH initiated the Humane Genome Project in 1990, with the goal to sequence the entire human genome by the year 2005. Stephens *et al*., 1990, *Science* 250:237; Cantor, 1990, *Science* 248:49-51. In order to achieve this goal within the projected time frame of fifteen years, 550,000 nucleotides of human DNA have to be sequenced and verified every single day. Once completed, the sequences of all the putative genes and their putative expression products, i.e., proteins, will be available for research scientists worldwide and will no doubt have a dramatic impact on the understanding of the molecular basis of human biology.

However, the vast amount of information which will be made available by the Human Genome Project will still be insufficient to resolve the mysteries behind most disease processes because cellular function or dysfunction results from the concerted interaction and differential expression of proteins. Indeed, the information resulting from the Genome Project will not provide any information as to when, where, and how much of a given gene is expressed.

In an attempt to obtain more meaningful information with respect to the expression profile of genes in the various cell or tissue types, several approaches have been developed which examine the levels of mRNA present within distinct cell types. Okubo *et al.,* 1992, *Nat. Genet.* 2:173-179; Velculescu *et al.,* 1995, *Science* 270:484-487; Liang and Pardee, 1995, *Curr. Opin. Immunol.* 7:274-280; Augenlicht *et al.,* 1987, *Cancer Res.* 47:6017-6021; Fodor *et al.,* 1993, *Nature* 364:555-556; Schena *et al.,* 1995, *Science* 270:467-470. In theory, the majority of mRNAs expressed within a cell would be translated into proteins; if one could catalogue the repertoire of mRNAs expressed, one could infer which proteins are expressed as well. Indeed, comparison of the expression levels of specific transcripts among different cell or tissue types, tissues or cells derived from different disease or developmental stages, or cells exposed to different stimuli has provided meaningful information with respect to particular genes' functions or their roles in the development of a disease. Approaches based on the determination of differences in the expression profiles of genes at the mRNA level have facilitated the identification of novel genes encoding products having a function of interest. Such approaches have permitted the identification of several genes, for example T cell receptor genes (Yanagi *et al*., 1984, Nature 308:145-149) and a number of tumor suppressor genes, including p21 (el-Deiry *et al.,* 1993, *Cell* 75:817-825; Noda *et al.,* 1994, *Exp. Cell. Res.* 211:90-98). Further, comparative assessment of relative amounts of nucleic acids has the potential to provide a valuable parameter for the organization of sequence information obtained through large scale sequencing approaches.

Others have used a so-called proteomics approach to understanding the expression profile of genes in cells. In proteomics, the expressed proteins themselves are analyzed, e.g., by two-dimensional acrylamide gel electrophoresis (2-DGE) of cellular extracts. Anderson and Anderson, 1994, *Electrophoresis* 17:443-453; Anderson *et al.,* 1982, *Trends in Analytical Chem.* 1:131-135; Anderson and Seilhamer, 1997, *Electrophoresis* 18:533-537. Recently it has become clear that, during the normal degradation and biosynthesis of all proteins within all cells, stable intermediates are formed before the conversion of the protein chain into single amino acids or functional protein molecules. Larsen and Finley, 1997, *Cell* 91:431-434; Gottesman *et al*., 1997, *Cell* 91:435-438; Coux *et al.,* 1996, *Annu. Rev. Biochem.* 65:801-847; Baumeister *et al.,* 1998, *Cell* 92:367-380.

### Summary of the Invention

The present invention generally relates to profiles of ligands which share the characteristic of being able to bind specifically to a particular multi-ligand binding receptor of a cell of interest. Generally these ligands are first obtained by extraction from a ligand/receptor complex, then further characterized and displayed or catalogued in a profile. The invention is based, in part, on the inventors' discovery that certain ligand-binding systems within a cell can be used to identify proteins expressed in that cell. Each system comprises one or more types of multi-ligand binding receptors that specifically bind cellular components present in a particular cell, e.g., peptides or proteins, in a highly reproducible manner, and as such the set of ligands bound to such multi-ligand receptors largely reflects the set of proteins expressed in that cell.

In particular, the power of the cell's multi-ligand binding receptor systems, including the MHC class I and MHC class II receptor systems, are harnessed to isolate and identify native ligands, e.g., proteins or stable peptide intermediates of protein degradation or biosynthesis, expressed within the cell of interest. The ligands so identified can be used to catalogue the proteins expressed and "turned over" in a cell for any particular cell type, metabolic state, etc. A characteristic profile or fingerprint of polypeptide ligands can be generated for a given cell type, for diseased vs. normal cells, for different metabolic or developmental states of a cell, etc. Appropriate comparisons of the profiles can be used to identify cellular targets useful in diagnostics, drug screening and development, and developing therapeutic regimens. Since the polypeptide ligands are representative of the set of proteins expressed by a given cell type, they can be termed "expressed protein tags" or "EPTs", conceptually similar to nucleic acid-based ESTs (expressed sequence tags).

More specifically, the invention is based, in part, on the inventors' discovery that multi-ligand receptors involved in a number of cellular metabolic and anabolic systems, including but not limited to the proteasome pathway, the ubiquitin pathway, cytosol/ER transport, antigen processing pathways, protein folding, protein unfolding, and protein trafficking, specifically recognize and bind proteins and stable intermediates, and as such can be used to extract and identify ligands, i.e., proteins and stable intermediates thereof, from a given cell of interest. The invention further relates to methods of generating such ligand profiles. The methods involve isolation of one or a plurality of multi-ligand receptors from a cell of interest, extraction of the ligands bound to the isolated receptor(s), and characterization of the so- isolated ligands according to a number of selected chemical or physical parameters, including molecular weight, amino acid sequence, and/or chemical nature such as charge or hydrophobicity

There is also described herein a stored database that includes three categories of data respectively representing (a) ligand profiles, (b) cell sources, and (c) multi-ligand binding receptor types (for brevity, referred to herein as "receptor types"). In the database, there are associations among the instances of the three categories of data. The database configures a computer to enable finding instances of data of one of the categories based on their associations with instances of data of another one of the categories.

Specifically, the cell sources may be based on cell types, cell conditions, particular individuals, states of perturbation, developmental states, or other criteria. The ligand profiles include information that uniquely identifies protein fragments, e.g., mass spectral data. The database may be queried (e.g., using a selected cell source having a selected cell condition) to find an instance of the ligand profiles that is associated with a selected one or more instances of the cell sources and a selected one or more instances of the receptor types. The found instances may include two ligand profiles that are compared to determine a difference between them.

Methods described herein include performing an experiment on cells, identifying a ligand profile associated with said cells, and, based on the ligand profile, querying a database that contains at least two categories of data, including ligand profiles and cell sources, to derive a cell source or a ligand profile and an associated cell source.

The experiment may have a variety of features. For example, the feature of the experiment may include treatment of the cells using a candidate drug regimen, and a cell source identified as a result of the query may represent a different treatment of cells (e.g., a different drug or use of the candidate drug in a different way).

The feature of the experiment may include treatment of an animal using a test compound regimen. The determined ligand profile may be associated with a given organ of the animal. A cell source identified as a result of the query may represent a different organ of an animal subjected to treatment using the test compound, or the same organ prior to treatment.

The feature of the experiment may include controlled cell development, and the determined ligand profile may be associated with the development of the cell. A cell source identified as a result of the query may be developmentally different from the cell source of the cells of the experiment.

The feature of the experiment may include introducing an expression vector into cells of a cell source, and the determined ligand profile may be associated with the effect of the expression vector on the cells.

The feature of the experiment may include response of cells to pharmacological compounds, and the determined ligand profile may be associated with responsiveness or non-responsiveness to the compound. The cell source identified as a result of the query may be phenotypically different from the cell source of the cells of the experiment.

In another method disclosed herein a cell source, a receptor type, or a ligand profile of interest is identified. Based on the identified cell source, receptor type, or ligand profile, a query is directed to a database that contains the three associated categories of data to derive information about cell sources, receptor types, or ligand profiles that relates to the cell source, receptor type, or ligand profile of interest.

In another method disclosed herein cells of a cell source are provided, a ligand profile is generated from the cells, and a query is directed to a database that contains the three associated categories to derive information about cell sources, receptor types, or ligand profiles that relates to the provided cell source and the generated ligand profile.

The invention affords a powerful approach for characterizing cellular proteins and other cellular components, and can be applied as a tool in a variety of settings including characterizing a cell type, analyzing the metabolic or developmental state of a cell, characterizing diseased vs. normal and cells, and identifying cellular targets involved in disease processes. In addition, the methods can be used to assist in mapping the genome and in functional genomics.

Terms used herein are in general as typically used in the art, unless otherwise indicated. The following terms are intended to have the following general meanings:
A "ligand profile" is an artificial (*i.e*., produced by the hand of man) representation of a set of ligands, wherein each ligand is separately represented in a manner that conveys information about one or more physical or chemical characteristics which in combination are sufficient to distinguish it from other ligands in the set. The term thus covers a simple list of ligands identified by amino acid sequence, by one or a series of other physical or chemical characteristics, or by code name, where that code name can be decoded to denote the distinguishing physical or chemical characteristic(s). The term also covers more complex, multi-dimensional representations such as the "fingerprint" defined below, and includes representations that exist solely in machine-readable form as well as those in a visualizable format. A profile is considered to be a reproducible characteristic of a cell if two identical experiments using identical cells produce essentially the same profile.
A "fingerprint" is a type of ligand profile, further characterized as a multi-dimensional plot of a specific set of ligands, where each axis of the plot represents a type of quantifiable physical or chemical attribute of the ligands (e.g., charge, hydrophobicity, size, etc.).
A "multi-ligand binding receptor" is a polypeptide molecule (or complex of polypeptide molecules) which does not contain nucleic acid and which reproducibly binds to a particular set of at least ten different proteins or peptides in or derived from a given animal cell, where the binding is noncovalent. The binding affinity is preferably less than about 10 *µ*M. Binding specificity is typically based on structural, chemical, or physical features, such as charge, length, hydrophobicity or hydrophilicity of side chains, amino acid composition, length of side chains, size, three-dimensional structure, etc. Multi-ligand binding receptors suitable for the practice of this invention typically bind a repertoire of ligands with a level of specificity and a level of stability that allows isolation of receptor/ligand complexes in a reproducible manner. Specific receptors that can be used include but are not limited to antibodies, antigen-binding fragments of antibodies, Major Histocompatibility Complex (MHC) class I receptors; MHC class II receptors; receptors involved in the folding and/or unfolding of proteins, such as heat shock proteins (Bukau *et al.,* 1998, *Cell* 92:351-366), chaperonins and chaperones (e.g., hsp100, hsp90, hsp70, hsp65, calnexin, calreticutin, BIP, grp96, and grp94 (Sallusto *et al*., 1995, *J. Exp. Med.* 182:389-400; Sandoval *et al.,* 1994, *Trends Cell. Biol.* 4:282-297)); mannosidase; and N-glycanase (Pfeffer *et al*., 1987, *Ann. Rev. Biochem.* 56:829-852). Other receptors are peptide transporters such as TAP, the 26S or 20S proteasome or its components, and receptors involved in the ubiquitin pathway, such as E2 carrier proteins, E3 ubiquitin ligases, and unfoldases; trafficking or retention proteins such as the KDEL receptor (Munro *et al.,* 1987, *Cell* 48:899); and the mannose receptor (Sallusto *et al*., 1995, *J*. *Exp. Med.* 182:389-400; Sandoval *et al*., 1994, *Trends Cell. Biol.* 4:282-297). Each of these receptors recognizes a plurality of different proteins or stable peptide intermediates thereof; thus, the polypeptides bound reflect a portion of the proteins expressed within the cell. The term multi-ligand binding receptor as used herein is intended to include any receptor fragment that comprises a multi-ligand binding domain of any of the above named receptors or receptor complexes, and thus which can function like a multi-ligand binding receptor in the methods of the invention. It also includes antibodies, or antigen-binding fragments thereof, if the antibodies are capable of binding to a plurality (typically at least 10, and preferably at least 50) of proteins or peptides produced by a given cell.
A "ligand", as that term is used herein, is a polypeptide at least 4 amino acids in length, which noncovalently binds to a multi-ligand binding receptor, as defined above, with an affinity that permits a receptor/ ligand complex to be isolated from the cell lysate, and then to be dissociated so that the ligand can be analyzed. This typically means an affinity of less than about 10 *µ*M, and preferably less than about 1 *µ*M. The ligand can be an intact protein or a fragment of a protein. The fragment can be, for example, an intermediate in the biosynthesis or degradation of the protein. Preferably, the ligand will be at least 5 amino acids in length, more preferably at least 6, e.g., at least 7, and most preferably at least 8. The term "protein" includes glycoproteins.
The term "ligands having distinct core peptides" refers to ligands no two of which have more than six consecutive amino acids in common. Thus, the term covers a set of two (or more) ligands which are, or are derived from, different proteins, or are derived from non- or slightly-overlapping parts of the same protein, so long as the sequences of the ligands do not overlap by more than six consecutive amino acids.
The term "cell source" refers to cells having a particular characteristic or characteristics. The characteristics may be expressed in terms of cell type, cell condition (e.g., normal or diseased), particular individuals from whom the cells were derived, state of perturbation, developmental state, metabolic state, or other criteria.

### Brief Description of the Drawings

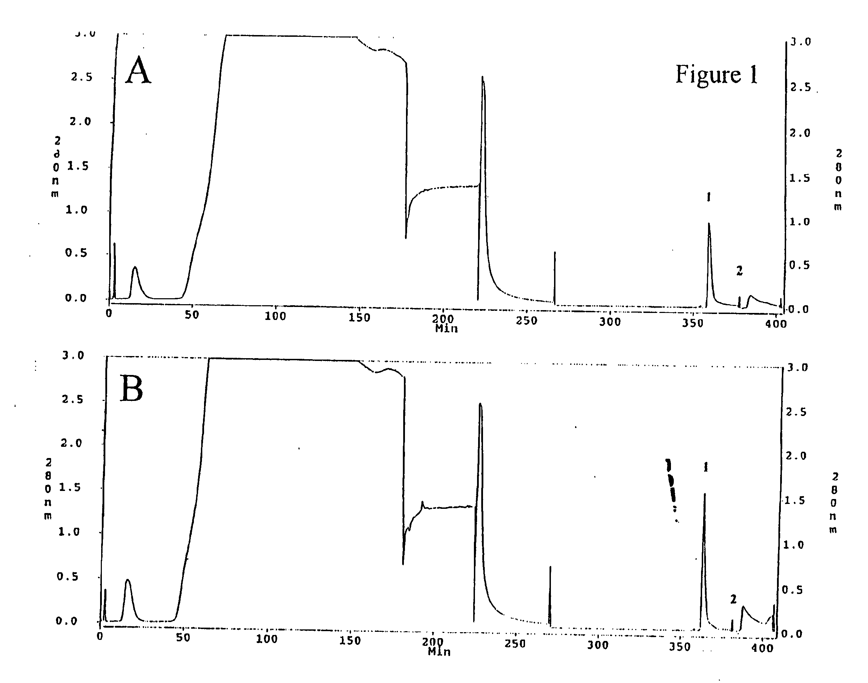

Figs. 1A and 1B are a pair of chromatograms illustrating a rapid and reproducible receptor:EPT complex purification of HLA-A*0201 and HLA-DR*0401/1301 from 20 g (Fig. 1A) and 22 g (Fig. 1B) of the human lymphoblastoid B cell line, JY, using an automated immunoaffinity chromatography purification strategy. The chromatograms represent the protein content as detected by UV absorbance at 280 nm on the y-axis and the time in minutes on the x-axis.

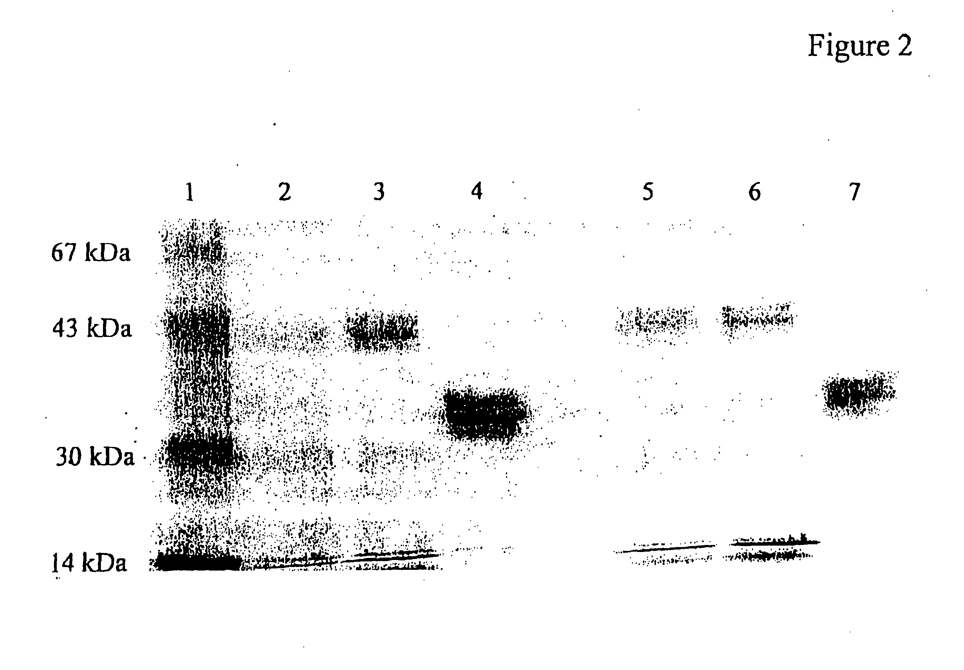

Fig. 2 is a photograph of an SDS-PAGE purity analysis of receptor:EPT complexes purified from the human B lymphoblastoid cell lines LG-2 and JY as shown in Figs. 1A and 1B.

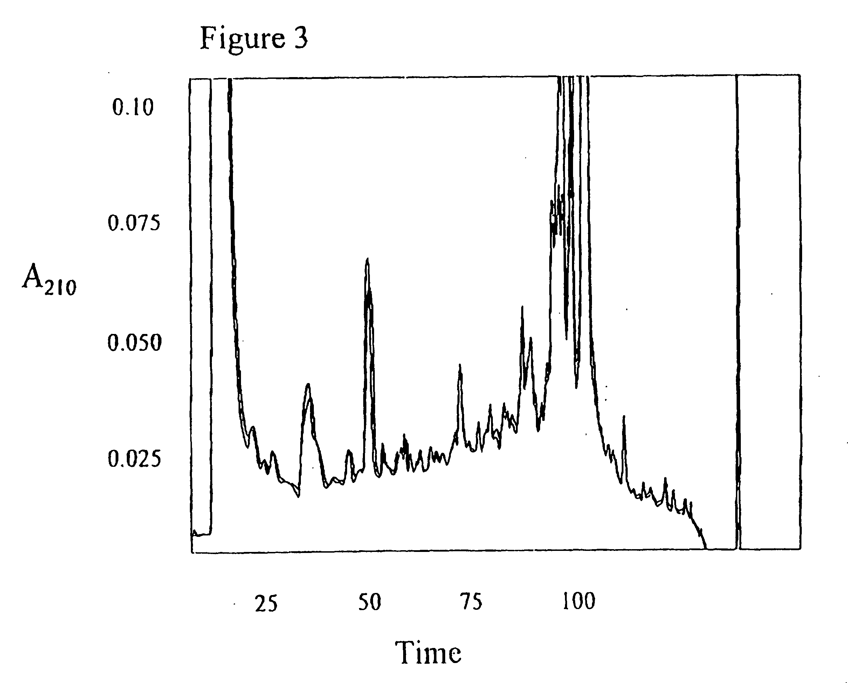

Fig. 3 is a pair of overlaid reversed-phase separation chromatograms of two independent HLA-A*0201:EPT preparations, as described in Figs. 1A and 1B. The two chromatograms represent the EPT repertoire as detected by UV absorbance at 210 nm and are overlaid to demonstrate the reproducibility of the separation necessary for EPT profile comparisons.

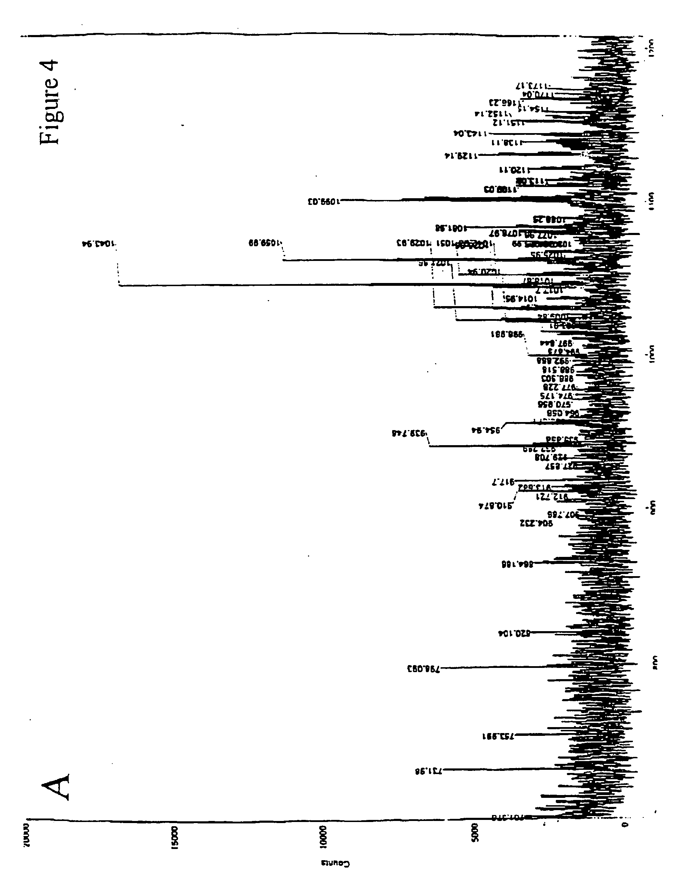

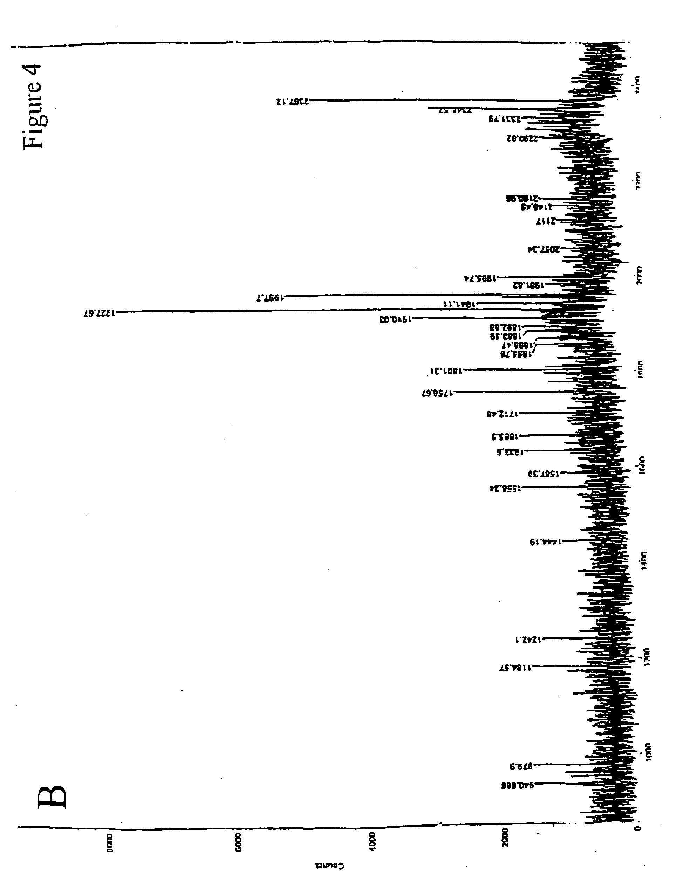

Figs. 4A and 4B are mass spectra analyses of single isolated fractions from two receptor:EPT preparations. Receptor:EPT isolation and EPT separation by reversed phase chromatography were carried out for HLA-A*0201 and HLA-DR*0401 from the human cell lines JY and Priess. Representative mass analyses for two EPT containing fractions are illustrated in Figs. 4A and 4B, respectively. The spectra represent the ionization of the complex mixture of individual EPTs contained in fractions 56 from the JY cell preparation (Fig. 4A) and 37 from the Priess cell preparation (Fig. 4B). The γ-axis displays the relative ionization of each EPT, and the x-axis displays the mass-to-charge ratio (m/z) for each charged species.

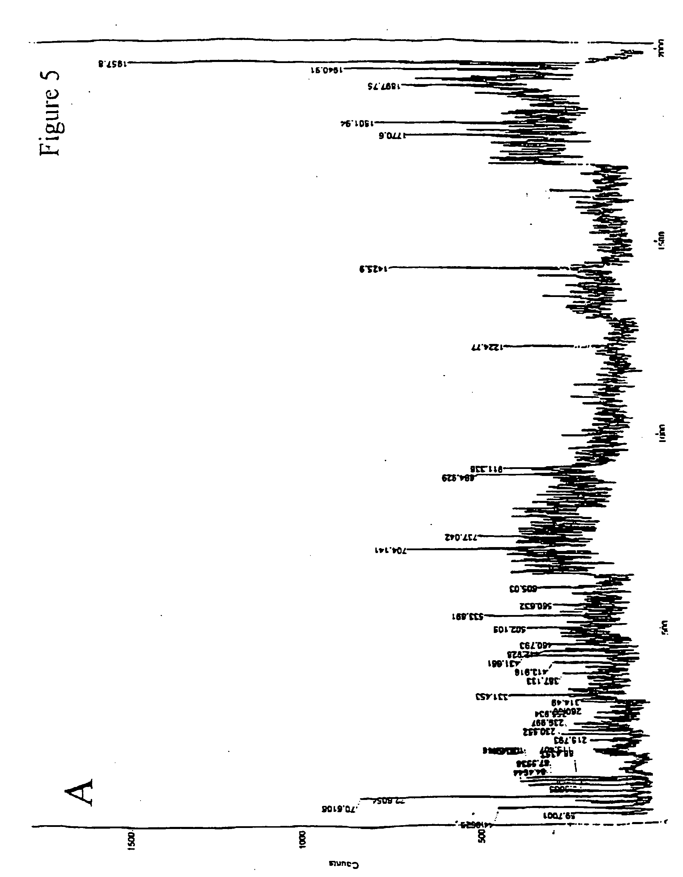

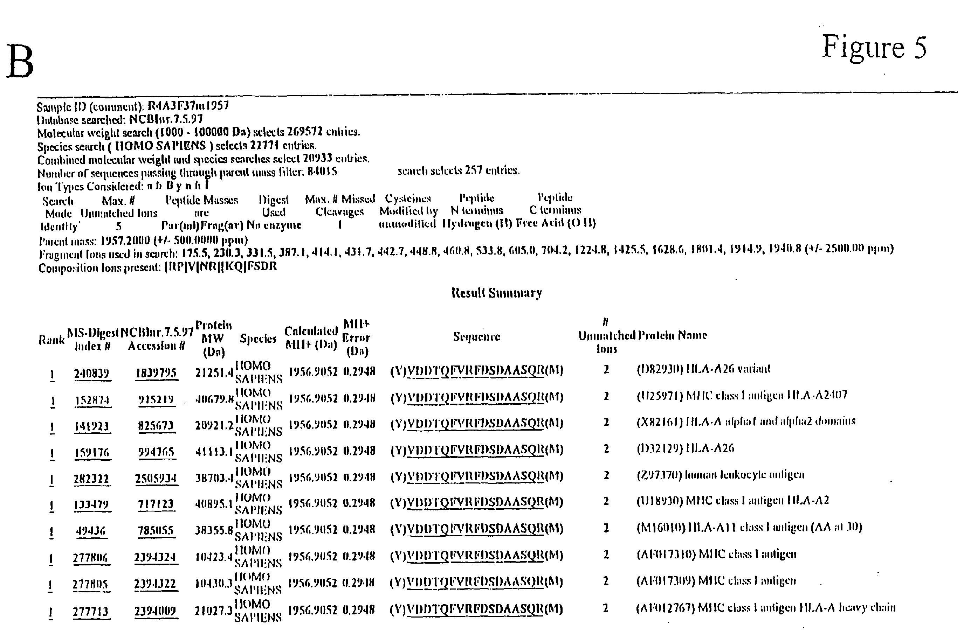

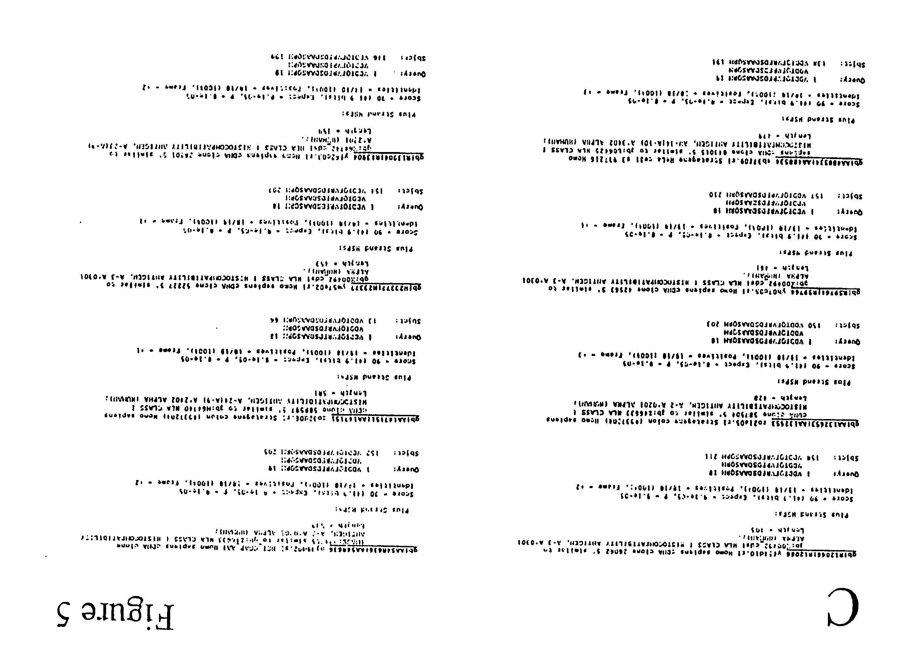

Fig 5A is a post-source decay/collisional-induced dissociation spectrum of an individual EPT from the analysis illustrated in Fig. 4B (m/z=1957.8). Fig. 5B is a table depicting a sequence analysis of that EPT based on the parent ion mass, the daughter ion fragments, and the immonium ion composition. Fig. 5C is a printout of the results of a search of the dbest database using the TBLASTN function from National Library of Medicine Genbank server to identify a corresponding EST in the database.

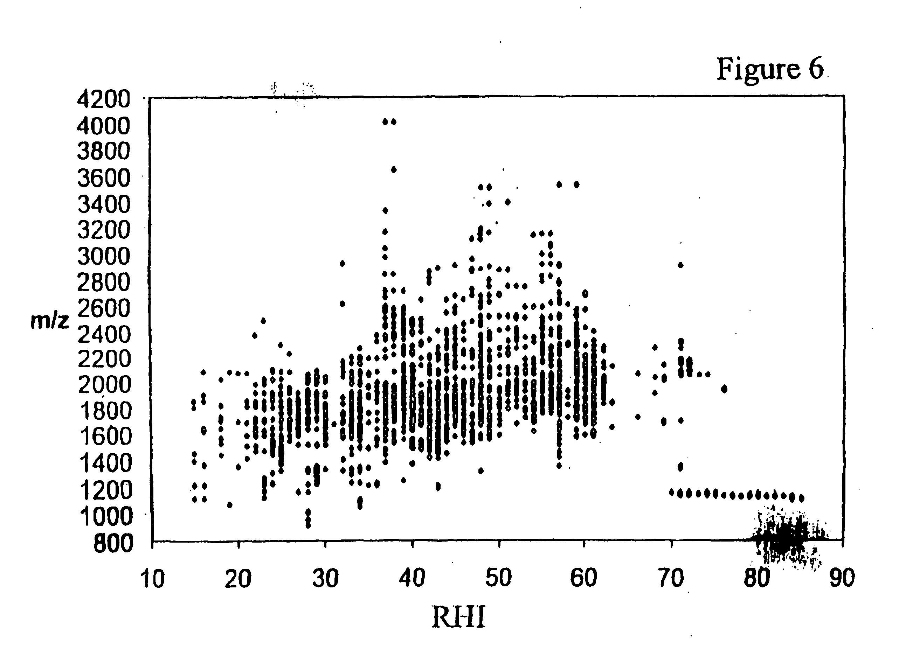

Fig. 6 is a two-dimensional EPT fingerprint for a human lymphoblastoid B cell illustrating EPTs extracted from the human receptor HLA-DR*1501. The Y axis displays mass-to-charge ratio (m/z), while the X axis displays relative hydrophobicity.

### Detailed Description of the Invention

The present invention relates, generally, to a novel approach to identifying, sorting, cataloguing, and/or profiling polypeptide molecules that are present in a given cell of interest. The invention is based, in part, on the inventors' discovery that internal systems present in each cell can be used as a tool for identifying and profiling the proteins expressed in a given cell. More specifically, the inventors found that promiscuous receptors, referred to as multi-ligand binding receptors, which are present within essentially each type of eukaroytic or prokaryotic cell and which bind a repertoire of ligands with high specificity and high affinity in a non-covalent fashion, can be used as a tool to extract ligands representing the protein repertoire, or a subset thereof, of a given cell of interest. Each cell has numerous distinct types of multi-ligand binding receptors, each of which binds ligands according to receptor-specific criteria. Isolating a specific multi-binding receptor from a cell of interest under conditions that preserve the receptor's association with its ligands allows for the identification of a subset of polypeptides specific for that particular cell. As different multi-ligand binding receptors bind different subsets of polypeptides, multiple subsets of polypeptides may be obtained by isolating different multi-ligand binding receptors from the same cell. The ligands may subsequently be extracted from the multi-ligand binding receptors to form a set of ligands which can be further characterized.

In accordance with the invention, a number of methods and tools can be used for cataloguing the isolated ligands according to specific parameters that allow assignment of a specific identity to each ligand. Such parameters include, but are not limited to, HPLC profiles, e.g., anion-exchange, cation-exchange, reversed-phase, normal phase, or hydrophobic-interaction chromatography; capillary electrophoresis profiles, e.g., CE, AEC-CE, CZE, or CEC-CE; and mass spectrometry profiles, e.g., MALDI-TOF/MS, FTMS, ESI-TOF, MALDI-ITMS, ESI-Quadropole MS, ESI-Quadropole/TOF-MS, ESI-Sector MS, FAB-MS, or ESI-ITMS. As such, the present invention allows for the generation of cell-specific profiles of ligands specifically binding to a selected multi-ligand binding receptor useful for the practice of this invention. The profiles of different cells, tissue or organ types of interest may be compared, and ligands may be identified that are differentially represented, e.g., present in one type of cell/tissue/organ, but absent from another, or expressed with different abundancy. Furthermore, "differential profiles" of ligands may be generated representing ligands which are differentially present in the two types of cells. Peptide and protein ligands represented in the profiles of the invention are referred to as "expressed protein tags" ("EPTs").

Thus, the invention includes a ligand profile which is characteristic for a given cell, the ligand profile containing a representation of at least ten different polypeptide ligands, all of which bind to a single type of multi-ligand binding receptor, wherein the representation either (1) characterizes each individual ligand based upon at least three physical or chemical attributes; or (2) characterizes each individual ligand based upon at least two physical or chemical attributes, one of these at least two attributes being mass or mass-to-charge ratio (with mass-to-charge ratio being defined as a single attribute); provided that, if the multi-ligand binding receptor is an MHC class I or class II receptor, at least 500 polypeptide ligands are represented in the ligand profile; and further provided that the ligand profile is a reproducible characteristic of the cell.

Alternatively, the ligand profile includes a representation of at least ten different polypeptide ligands, all of which bind to a single type of multi-ligand binding receptor, wherein the representation characterizes each individual ligand based upon at least one physical or chemical attribute, the at least one physical or chemical attribute comprising amino acid sequence; provided that, if the multi-ligand binding receptor is an MHC class I or class II receptor, at least 50 polypeptide ligands are represented in the ligand profile; and further provided that the ligand profile is a reproducible characteristic of the cell.

Also within the invention is a ligand profile which is characteristic for a given cell, the ligand profile comprising ion fragmentation patterns for at least ten different polypeptide ligands, all of which polypeptide ligands bind to a single type of multi-ligand binding receptor; provided that, if the multi-ligand binding receptor is an MHC class I or class II receptor, at least 100 polypeptide ligands are represented in the ligand profile; and further provided that the ligand profile is a reproducible characteristic of the cell.

In another embodiment, the invention includes a ligand profile which is characteristic for a given cell, the ligand profile comprising amino acid sequences of at least ten different polypeptide ligands having distinct core peptides, all of which ligands bind to a single type of multi-ligand binding receptor; provided that, if the multi-ligand binding receptor is an MHC class I or class II receptor, at least 100 polypeptide ligands (and preferably 150, 200, 300, or 500) are represented in the ligand profile; and further provided that the ligand profile is a reproducible characteristic of the cell.

In any of the above aspects of the invention, the multi-ligand binding receptor can be a MHC class I or MHC class II receptor, or can be a protein or multi-protein complex that is not an MHC class I or MHC class II receptor: e.g., a chaperone, a chaperonin, a calnexin, a calreticutin, a mannosidase, a N-glycanase, a BIP, a grp94, a grp96, hsp60, hsp65, hsp70, hsp90, hsp25, an E2 ubiquitin carrier protein, an E3 ubiquitin ligase, an unfoldase, hsp100, a proteasome, a trafficking protein, or a retention protein. The cell can be a hematopoietic cell (e.g., derived from blood or bone marrow) such as a B cell, or any type of cell other than a B cell. Useful physical or chemical attributes include charge, mass-to-charge ratio, size, hydrophobicity, and amino acid sequence. When the attributes include hydrophobicity and mass-to-charge ratio, they are typically determined using mass spectroscopy. The ligand profile can be combined with a second ligand profile, the second ligand profile (a) also being a reproducible characteristic of the given cell, and (b) containing a representation of at least ten additional polypeptide ligands, all of which bind to a second type of multi-ligand binding receptor different from the first type of receptor. If desired, these can be combined with any number of other such ligand profiles which are reproducible characteristics of the given cell, all derived from different types of multi-ligand binding receptors, to give more complete and detailed information about the set of proteins expressed by the given cell.

Also within the invention is a method of generating a reproducible ligand profile for a given cell type, which cell type comprises a selected type of multi-ligand binding receptor, the method including the following steps (with steps (f) - (k) being for the purpose of confirming the reproducibility of the profile generated in steps (a) - (e)):
(a) providing a first sample of the given cell type, wherein the first sample includes a first plurality of polypeptide ligands bound to the selected type of multi-ligand binding receptor;
(b) isolating the selected type of multi-ligand binding receptor from the first sample;
(c) separating the first plurality of ligands from the selected type of multi-ligand binding receptor;
(d) fractionating the first plurality of ligands;
(e) generating a first profile distinguishing among the first plurality of ligands on the basis of at least one chemical or physical attribute;
(f) providing a second sample of the given cell type, the second sample being essentially identical to the first sample, wherein the second sample comprises a second plurality of polypeptide ligands bound to the selected type of multi-ligand binding receptor;
(g) isolating the selected type of multi-ligand binding receptor from the second sample;
(h) separating the second plurality of ligands from the selected type of multi-ligand binding receptor;
(i) fractionating the second plurality of ligands;
(j) generating a second profile distinguishing among the second plurality of ligands on the basis of the at least one chemical or physical attribute; and
(k) confirming that the first profile and the second profile are essentially identical, and together represent a reproducible ligand profile for the given cell type.

In such a method, as in the related methods described below, a second, third, or additional chemical or physical attribute of each ligand can also be determined subsequent to the fractionation steps, and then represented in the profiles. The isolating and separating steps for all of the disclosed methods can be conveniently accomplished using appropriate columns arranged in an in-line system. In such an in-line HPLC system, chromatographic columns are arranged in series to allow continuous flow of the mobile phase from one column to the next, without removal from the system between columns. If desired, immunoaffinity columns, ion exchange chromatography columns, and/or ConA chromatography columns may be used for the isolating steps, while the next stage (e.g., reversed-phase chromatography) may be used for the fractionating steps, with each profile reflecting the relative time of elution of each ligand from the chosen chromatographic column. For example, the profile can include for each ligand a plot of the time of elution from the substrate vs. the mass-to-charge ratio.

Further information can be obtained if the method produces a profile or set of profiles that represents ligands derived from two or more types of multi-ligand binding receptors in the given cell type, e.g. by carrying out the following steps:
(a) providing a sample of lysate of the given type of cell, wherein the sample comprises a first plurality of polypeptide ligands bound to a first type of multi-ligand binding receptor and a second plurality of polypeptide ligands bound to a second type of multi-ligand binding receptor;
(b) isolating the first and second types of multi-ligand binding receptors from the sample;
(c) separating the first plurality of ligands from the first type of multi-ligand binding receptor and the second plurality of ligands from the second type of multi-ligand binding receptor;
(d) fractionating the first plurality of ligands and the second plurality of ligands; and
(e) generating a first profile distinguishing among the first plurality of ligands on the basis of at least one chemical or physical attribute and a second profile distinguishing among the second plurality of ligands on the basis of the same at least one chemical or physical attribute.

The techniques can be used to compare one cell preparation to another by generating a subtraction profile of polypeptide ligands, comprising:
(a) producing a first ligand profile by a method comprising:
   (i) providing a first sample comprising a first cell of interest, wherein the first cell of interest comprises a given type of multi-ligand binding receptor bound to a first set of polypeptide ligands;
   (ii) isolating the given type of multi-ligand binding receptor and the first set of ligands from the first sample;
   (iii) separating the first set of ligands from the given type of multi-ligand binding receptor;
   (iv) generating a first profile distinguishing among the first set of ligands on the basis of at least one chemical or physical attribute;
(b) producing a second profile of ligands by a method comprising:
   (i) providing a second sample comprising a second cell of interest, wherein the second cell of interest comprises the given type of multi-ligand binding receptor, bound to a second set of polypeptide ligands;
   (ii) isolating the given type of multi-ligand binding receptor and the second set of ligands from the second sample;
   (iii) separating the second set of ligands from the given type of multi-ligand binding receptor;
   (iv) generating a second profile distinguishing among the second set of ligands on the basis of the same at least one chemical or physical attribute;
(c) comparing the first profile and the second profile to identify differentially expressed ligands, thereby forming a subtraction profile of ligands. The first cell sample and the second cell sample may be obtained from different types of biological tissue (e.g., comparing smooth muscle tissue to skeletal muscle tissue), different cell types (e.g., endothelial cells and epithelial cells), different organ systems (e.g., pancreas and lung), or the same organ system but cells of different status (e.g., terminally differentiated vs. embryonic, or healthy vs. diseased or predisposed to a disease). Alternatively, the methods can compare transfected cells which express a particular recombinant nucleic acid vs nontransfected cells or transfected cells which do not currently express the recombinant nucleic acid. The methods could also compare cells treated in a particular way (either *<i>in vivo <*/*i>* or *in vitro)* vs. cells treated in a different way, or untreated. For example, the treatment may involve administration of a test substance or drug candidate such as a growth factor, a hormone, a cytokine, a small molecule, a polypeptide, a nucleic acid, a carbohydrate, or a lipid. Alternatively, the treatment may involve exposing the cells to stress conditions such as trauma, hypoxia, deprivation of glucose, deprivation of an amino acid, deprivation of a nutrient, presence of a toxin, or low or high temperature. The cells for any of these methods are preferably vertebrate cells (e.g., from a bird or fish), and more preferably mammalian cells, e.g., from a human or from a non-human animal such as a non-human primate, a mouse, rat, guinea pig, hamster, rabbit, dog, cat, cow, horse, pig, sheep, or goat. By adding another series of steps similar to (a) (i) - (iv) using a third cell sample, one could compare three different cell samples, or compare the first sample to the second and to the third. For example, the second cell sample could be a positive control and the third cell sample a negative control, or the three cell samples could represent three different treatment regimens.

In a variation on the above, one can simply compare the proteins expressed in a first cell sample to those expressed in a reference cell sample, by generating a ligand profile that is compared to an appropriate reference ligand profile, as follows:
(a) producing a first ligand profile by a method comprising:
   (i) providing a first cell sample comprising a given type of multi-ligand binding receptor bound to a first set of polypeptide ligands;
   (ii) isolating the given type of multi-ligand binding receptor and the first set of ligands from the first cell sample;
   (iii) separating the first set of ligands from the given type of multi-ligand binding receptor;
   (iv) generating a first ligand profile distinguishing among the first set of ligands on the basis of at least one chemical or physical attribute;
(b) providing a reference ligand profile representing a second set of polypeptide ligands extracted from the given type of multi-ligand binding receptor of a reference cell sample (e.g., a sample which contains diseased cells of an animal, or cells treated or not treated with a particular compound), wherein the reference ligand profile distinguishes among the second set of polypeptide ligands on the basis of the at least one chemical or physical attribute; and
(c) comparing the first ligand profile to the reference ligand profile, in order to identify differences or similarities between the first cell sample and the reference cell sample. This and the other comparison methods described above can be used to compare, for example, cells cultured in the presence of a test compound to cells not cultured in the presence of the test compound; or cells from an animal treated with a test compound to cells (1) from the same animal before the treatment, or (2) from a second animal not treated.

Also within the invention is a set of ligand profiles, the set including
(a) a first ligand profile comprising a first representation of a first plurality of polypeptide ligands, all of which bind to at least one multi-ligand binding receptor of a first cell, wherein the first representation distinguishes among the members of the first plurality of ligands based upon at least one physical or chemical attribute; and
(b) a second ligand profile comprising a second representation of a second plurality of polypeptide ligands, all of which bind to the at least one type of multi-ligand binding receptor of a second cell, wherein the second representation distinguishes among the second plurality of ligands based upon the at least one physical or chemical attribute;
provided that (i) the first cell differs from the second cell in a parameter selected from the group consisting of genetic background, culture conditions, genetic background plus culture conditions, <i>in vivo </i> exposure to a test compound, and genetic background plus <i>in vivo </i> exposure to a test compound; and (ii) any significant difference between the first and the second ligand profiles is attributable to that parameter. Such a set can include, of course, additional profiles which differ from the above first and second profiles in that they are derived from other cell sources. In addition, the set can include other profiles representing ligands extracted from the same cell sources as above, but using a different multi-ligand binding receptor in order to give more complete information about the proteins expressed in the cells.

The invention can be used in a method of detecting a difference between the set of proteins expressed in a first cell and the set of proteins expressed in a second cell, which method includes
(a) providing a first ligand profile made by a method involving the steps of:
   (i) providing a first cell which contains at least one type of multi-ligand binding receptor, bound to a first set of polypeptide ligands,
   (ii) isolating from the first cell the at least one type of multi-ligand binding receptor bound to the first set of ligands,
   (iii) separating the first set of ligands from the at least one type of multi-ligand binding receptor, and
   (iv) generating a first ligand profile distinguishing among the members of the first set of ligands on the basis of at least one chemical or physical attribute;
(b) providing a second ligand profile made by a method involving the steps of:
   (i) providing a second cell comprising the at least one type of multi-ligand binding receptor, bound to a second set of polypeptide ligands,
   (ii) isolating from the second cell the at least one type of multi-ligand binding receptor, bound to the second set of ligands,
   (iii) separating the second set of ligands from the at least one type of multi-ligand binding receptor, and
   (iv) generating a second ligand profile distinguishing among the members of the second set of ligands on the basis of the at least one chemical or physical attribute;
(c) comparing the first ligand profile to the second ligand profile, in order to identify any difference between the first and second profiles, wherein such a difference is an indication of a difference between the set of proteins expressed in the first cell and the set of proteins expressed in the second cell. If desired, one can perform either or both of the following additional steps:
   (i) selecting a ligand which is represented in one profile but not in the other, and identifying the amino acid sequence of the ligand; and/or
   (ii) generating a differential profile which sets forth at least some of the differences between the set of proteins expressed in the first cell and the set of proteins expressed in the second cell. Such a differential profile is also considered to be within the invention.

Once at least part of the amino acid sequence of a ligand is determined, the sequence of the full protein can be determined (either by searching for a match in a sequence database, or by using degenerate probes to clone a cDNA encoding the full protein). If desired, an expression vector encoding the protein can then be prepared and used to study the role of the expressed protein in the cell, e.g. as a target for drug development.

Since most types of cells express MHC class I constitutively, and the expression of MHC class II receptors can be induced in many cell types with cytokines such as gamma-interferon, these are both excellent candidates for the multi-ligand binding receptors utilized in the methods and profiles of the invention.

Based on the above, the invention relates, in more specific embodiments, to a unique approach for generating libraries and profiles of EPTs that can be used to identify, catalogue and characterize most or all proteins expressed within a cell for any given cell type, metabolic or developmental stage, and disease vs. normal state, or in response to a test substance such as a given hormone, growth factor, transcription factor, cytokine, small molecule, polypeptide, nucleic acid, carbohydrate or lipid. The approach can also identify differences between transgenic vs. non-transgenic cells, or transfected vs. non-transfected cells. As such, the invention relates to the identification of "ligand profiles" of a cell type of interest. These profiles can be used to pre-sort cellular proteins for "proteomics" analysis, greatly reducing the screening effort and increasing the efficiency of identifying cellular proteins involved in developmental and metabolic disease processes. Appropriate comparisons of the profiles can be used to identify cellular targets useful in diagnostics, drug screening and development, and for developing therapeutic regimens.

In short, the invention provides a "snapshot" of the proteins expressed and turned-over within a given cell by the generation of EPT profiles, and the cataloguing, identification and isolation of proteins differentially expressed in two or more populations of cells; such data will facilitate the identification of proteins that have biological significance to a particular cellular state, e.g., in metabolism, maturation, development, disease or treatment.

Generally, every multi-ligand binding receptor present in a cell that recognizes specific polypeptides produced by that cell and fulfills certain requirements that are listed below is intended to be within the scope of this invention. Numerous multi-ligand binding receptors that bind polypeptide components specifically produced by a given cell will give insight into cell-specific protein expression; developmental, anabolic or metabolic processes; or other aspects of the biology and physiology of a given cell, tissue type, or organ system. Multi-ligand binding receptors within the scope of the invention, and useful for the practice of the invention, include but are not limited to receptors involved in various protein biosynthesis and degradation pathways. They typically bind to their repertoire of ligands with high specificity and in a highly discriminatory manner. Typically, the ligands are, e.g., cellular proteins, or intermediates of protein biosynthesis or degradation (i.e., peptides). For the practice of the invention, it is critical that (1) the repertoire of ligands is bound with high specificity and affinity, and (2) the receptor/ligand complex is sufficiently stable so that when the receptor is isolated, the bound ligands remain reproducibly associated with the receptor. Preferably, the multi-ligand binding receptors used as tools for generating the libraries and profiles of the present invention have a receptor/ligand affinity of less than about 10 *µ*M, more preferably of less than about 1 *µ*M, and most preferably of less than about 100 nM. Furthermore, each receptor recognizes a signal on the ligand that may be based on structural, chemical, or physical features, such as charge, length, hydrophobicity or hydrophilicity of side chains; amino acid composition or sequence; size; or three-dimensional structure.

It is well established that cellular protein biosynthesis involves enzymatic modifications that require binding of the intermediates to receptors. For example, chaperones are a class of protein intermediate binding receptors that recognize and bind their substrates based on their stage of folding during protein maturation. Generally, chaperones are present in each cellular compartment in which proteins must fold, *i.e*., the cytosol, the nucleus, the mitochondria, chloroplasts, lysosomes, and the endoplasmatic reticulum (ER). For review, *see*, Melnick and Argon, 1995, *Immunology Today* 16:243-250. Examples of chaperones include BiP (for binding protein), also known as GRP78, located in the lumen of the ER and a member of the heat shock protein 70 family of stress proteins (Nakaki *et al*., 1989, *Mol. Cell. Biol.* 9:2233-2238); GRP96 (for glucose-regulated protein 96); GRP94 (for glucose-regulated protein 94), also known as ERp99; endoplasmin; gp96; hsp100, a ER member of the hsp90 family of stress proteins (Lee, 1993, *Trends Biochem. Sci.* 12:20-23; Mazarella and Green, 1987, *J. Biol. Chem.* 262:8875-8883; Koch *et al*., 1986, *J. Cell Science* 86:217-232; Li and Srivastave, 1993, *EMBO J.* 12:3143-3151; Sargan *et al.,* 1986, *Biochemistry* 25:6252-6258); calnexin, also known as p88; IP90, a Ca²⁺-binding phosphoprotein that associates with the ER translocation machinery and is related to calreticulin (Ou *et al*., 1993, *Nature* 364:771-776); and calreticulin (Degen *et al*., 1992, *J*. *Exp. Med.* 175:1653-1661).

Another group of multi-ligand binding receptors involved in protein biosynthesis pathways includes a number of cytosolic receptors involved in the translocation and folding of nascent proteins. Neupert and Lill, 1994, *Nature* 370:421-422; Frydman *et al.,* 1994, *Nature* 370:111-117; Bukau and Horwich, 1998, *Cell* 92:351-366. For example, *hsps* are thought to recognize, interact with and facilitate maturation of a number of newly synthesized proteins. For review, see, Welch, 1992, *Physiological Reviews* 72:1063-1081. It follows that hsps recognize and bind to a number of preselected proteins in a cell, and as such provide a powerful tool for the practice of this invention. Specific examples of such cytosolic multi-ligand binding receptors include another set of chaperones, including hsp70s (Flynn *et al.,* 1991, Nature 353:726-730; Landry *et al.,* 1992, Nature 355:455-457; Blond-Elguindi et al., 1993, *Cell* 75:717-728; Lewis and Pelham, 1985, *EMBO J.* 4:3137-3142; Flynn *et al.,* 1989, *Science* 245:385-390), which are thought to prevent the premature folding and aggregation of polypeptides during membrane translocation and translation; hsp60s or chaperonins (Hemmingsen *et al*., 1988, Nature 333:330-334), which are large oligomeric complexes mediating the folding of polypeptide chains in an ATP-dependent reaction (Goloubinooff *et al.,* 1989, *Nature* 342:884-889; Martin *et al.,* 1991, *Nature* 352:36-42); CCT/TRiC (Horwich and Willison, 1993, *Phil. Trans. R. Soc.* 339:313-325); and hsp40 (Neupert and Lill, *supra*).

Another group of multi-ligand binding receptors involved in protein biosynthesis pathways includes a number of post-translational modification enzymes, such as the ER and cis-Golgi resident mannosidase and N-glycosidases (Pfeffer *et al.,* 1987, *Ann. Rev. Biochem.* 56:829-852), and trafficking or retention proteins, such as the KDEL receptor (Munro *et al*., 1987, *Cell* 48:899) and the mannose receptor (Sallusto *et al*., 1995, *J*. *Exp. Med*. 182:389-400; Sandoval *et al*., 1994, *Trends Cell. Biol*. 4:282-297).

A second general category of multi-ligand binding receptors useful for the practice of this invention includes receptors involved in cellular degradation pathways of proteins (Hochstrasser, 1996, *Cell* 84:813-815; Hasselgren and Fischer, 1997, *Ann. Surg.* 225:307-316). It is well established that intracellular proteins, once synthesized, are continually degraded back to their constituent amino acids. In recent years, a clearer picture of the degradative pathways and proteolytic machinery involved, and their biological significance, has been elucidated. It is now known that most cellular proteins are hydrolyzed by a soluble ATP-dependent system that is present in both the nucleus and the cytosol (Ciechanover, 1994, *Cell* 79:13-21). Often, protein substrates are first marked for degradation by covalent conjugation to multiple molecules of a small protein, ubiquitin. (Ciechanover, 1994, supra.) This process involves the activation of ubiquitin by the formation of a thiol-ester at its carboxyl terminus, which is then transferred to the ε-amino group on a lysine residue on the protein. Other ubiquitin molecules are progressively linked to the first, forming long chains of ubiquitin on the substrate. This triggers the rapid hydrolysis of the protein substrate by a very large ATP-dependent proteolytic complex, termed the 26S proteasome. See, for example, Goldberg, 1995, *Science* 268:522-523; Peters, 1994, *Trends Biochem. Sci.* 19:377-382; Rubin and Finley, 1995, *Curr.* Biol. 3:854-858; Goldberg and Rock, 1992, Nature 357:375-379; Goldberg et al., 1995, *Current Biology* 2:503-508; Rock *et al*., 1994, *Cell* 78:761-771; Fenteany *et al*., 1995, *Science* 268:726-730; Read *et al.,* 1995, *Immunity* 2:493-506. The physiological role of the proteasome is believed to be at least three-fold. First, the proteasome has an important function in the degradation of damaged or mutated cellular proteins. Bukau and Horwich, 1998, *Cell* 92:351-366. Second, the proteasome appears to play an essential role in the degradation of various regulatory proteins (Ciechanover, 1994, *supra*). Rapid removal of such proteins is necessary for the control of cell growth and metabolism. For example, the orderly progression of cells through the mitotic or meiotic cycle requires the programmed ubiquitination and destruction of the various cyclins via CDC34 or the cyclosome pathway (King et. al, 1996, *Science* 274:1652-1659; Glotzer, 1991, *Nature* 349:132-138; Scheffner *et al.,* 1993, *Cell* 75:495-505; Chen *et al.,* 1996, *Biochemistry* 35:3227-3237). Third, the proteasome has been shown to have a distinct role in the processing of antigens for presentation to T-lymphocytes.

More specifically, certain binding and recognition proteins of the proteasome pathway are useful as multi-ligand binding receptors for the purpose of the invention. Particularly useful tools for this approach are a number of different multi-ligand binding receptor types present in the ubiquitin-proteasome pathway for protein degradation. (Scheffner *et al.,* 1993, *Cell* 75:495-505; Chen *et al.,* 1995, *Genes and Development* 9:1586-1597; Hochwasser, 196, *Cell* 84:813-815.) These include, but are not limited to, ubiquitin-conjugating enzymes (E2s) (Jentsch *et al*., 1991, *Biochim. Biophys. Acta* 1089:127-139; Quin *et al*., 1991, *J. Biol. Chem.* 266:15549-15554), including but not limited to CDC34; and ubiquitin-protein ligases (E3s) (Hershko and Ciechanover, 1992, *Annu. Rev. Biochem.* 61:761-807), including but not limited to the cyclosome and its components (King *et al.,* 1996, *Science* 274:1652); G1/SKP1/Cullin/F-box complex (King *et al.,* 1996, *supra);* E3α (Hershko and Ciechanover, 1992, *supra);* hectdomain proteins (Kumar et *al.,* 1997, *J. Biol. Chem.* 272:13548-13554; Plant *et al.,* 1997, *J. Biol. Chem.* 272:32329-32336; Huibregtse *et al*., 1997, *Proc*. *Natl. Acad. Sci. USA* 94:3656) or ligand-binding components thereof; unfoldases (Lupaset *et al.*, 1993, *Enz. Prot.* 47:252-273); the 26S proteasome complex (Rechsteiner et al., 1993, J. *Biol.* Chem. 268:6065-6068; Peters *et al*., 1993, *J. Mol. Biol.* 234:932-937) or ligand-binding components thereof; the 20S proteasome complex (Peters et *al.,* 193, supra) or ligand-binding components thereof; and the ER resident UBC6 and UBC7 (ubiquitination degradation enzymes) (Sommer and Jentsch, 1993, *Nature* 365:175-179*;* Jentsch, 1992, *Annu. Rev*. *Genet*. 26:179-207).

Other MLRs include heat shock proteins (hsp), which are involved in the implementation of a cell's response to stress conditions, such as changes in their normal growth temperature, metabolic insults, various heavy metals, agents that modify sulfhydryls, various ionophores, and a number of other metabolic agents.

Thus, a wide variety of different multi-ligand binding receptors may be used to practice the present invention. Depending on the specific experimental question involved, a given multi-ligand binding receptor system may be preferred. For example, if it is desired to identify a profile of the protein repertoire expressed by a specific cell or tissue type, typically a multi-ligand receptor system (or a combination of several systems) will be employed that captures a large array of ligands, reflecting as many of the expressed cellular proteins as possible. Suitable multi-ligand binding receptor systems for this sort of task include MHC class I and MHC class II receptors (most preferably a combination of several allotypes), which are believed to present peptides derived from virtually every cellular protein. (Kourilsky *et al*., 1987, *Proc. Natl. Acad. Sci. USA* 84:3400-3404; Claverie and Kourilsky, 1986, *Ann*. *Inst. Pasteur Immunol.* 137D(3):425-442; Kourilsky and Claverie, 1986, *Ann. Inst. Pasteur Immunol.* 137D (1) :3-21.) One the other hand, if it is desired to determine whether a specific set of ligands is differentially expressed, e.g., present or absent in a cell or tissue type, for example after treatment with a certain substance of interest, a multi-ligand binding receptor system specifically recognizing that set of ligands can be employed. Thus, for example, if the question involves how a chemical compound affects the cell cycle, the multi-ligand binding receptor system chosen may be the cyclosome or a component thereof. Or, as another example, if it is desired to isolate ligands and/or generate a ligand profile of secretory monomeric glycoproteins expressed in a given cell, calnexin would be a multi-ligand binding receptor of choice (Ou *et al*., 1993, *Nature* 364:771-776). The skilled artisan will be able to determine which multi-ligand binding receptor system, or combination of several receptor systems, is most suitable for any specific application. The following description will focus and elaborate primarily on multi-ligand binding receptors which are part or auxiliaries of the MHC receptor systems, which appear to be particularly well suited for generation of EPT profiles of a cell of interest, as, with few exceptions, each and every protein of a given cell is believed to be recognized by MHC receptors. However, the invention is not intended to be limited to such; the skilled artisan will be able to adapt the described protocols for practicing the invention with any other suitable multi-ligand binding receptor within the scope of the invention. *See, supra.*

In preferred embodiments of the invention, the multiple-ligand binding receptors used are MHC class I and MHC class II receptors. In humans they are referred to as HLA receptors, and in mice they are referred to as H-2 receptors; the homologous systems of other species may be referred to by other terminology (*e.g.,* BoLA as the cattle MHC homologue, *see*, Gaddum *et al*., 1996, *Immunogenetics* 43:238-239; DLA as the canine homologue, *see,* Wagner *et al., Tissue Antigens* 48 :549-553). MHC class I and MHC class II receptors are particularly attractive for practicing the invention because, among their several isotypes, they are believed to bind stable peptide intermediates of most proteins present in a given cell. Researchers in the field of immunology have previously isolated and characterized some of the peptides bound to members of the MHC family of receptors (Harris *et al.,* 1993, *The Journal of Immunology* 151:5966-5974; Chicz *et al.,* 1993, *J. Exp. Med.* 178:27-47; Chicz et *al., J. Immunol.* 159:4935-4942; Chicz *et al*., 1994, *International Immunology* 6:1639-1649; Chicz et *al.,* 1992, *Nature* 358:764-768; Davenport *et al*., 1995, *Proc. Natl. Acad. Sci. USA* 92:6567-6571; Urban et *al.,* 1994, *Proc. Natl. Acad. Sci. USA* 91:1543-1538). Human class I and class II MHC molecules comprise at least nine major subtypes, i.e., HLA-A, HLA-B, HLA-C, HLA-E, HLA-F, and HLA-G for MHC class I, and HLA-DR, HLA-DQ, and HLA-DP for MHC class II (Urban *et al.,* 1993, *Chem. Immunol.* 57:197-234; Trowsdale *et al.,* 1991, *Immunology Today* 12:443). Multiple alleles have been described for each isotype, with HLA-DR categorized as the most polymorphic (at least two DRα and at least 221 DRβ alleles), followed by HLA-DQ (at least 18 DQα1 and at least 31 DQβ1 alleles), and HLA-DP (at least 10 DPα1 and 77 DPβ1 alleles). Bodmer *et al*., 1996, *Tissue Antigens* 49:297. Class I alleles consist of a non-polymorphic β2 microglobulin (light chain) associated with a polymorphic heavy chain. HLA-A has been described to comprise at least 83 allotypes, HLA-B has been described to comprise at least 186 allotypes, and HLA-C has been described to comprise at least 42 allotypes. Bodmer *et al*., 1997, *Tissue Antigens* 49:297-321.

The different isotypes and alleles have been shown to bind distinct but overlapping sets of peptides. Chicz *et al*., 1993, *J. Exp. Med.* 178:27-47. Virtually every mammalian cell expresses MHC isotypes, which present distinct peptides reflecting the cell's protein content on the cell surface. Both extracellular "foreign" antigens, taken up by the cell through phagocytosis, and intracellular "self" proteins are degraded by the proteasome pathway, and transported from the cytosol to the TAP1/TAP2 transporter (Rock *et al.,* 1994, *Cell* 78:761-771; Goldberg and Rock, 1992, *Nature* 357:375-379; Momburg *et al*., 1996, *in*: MHC Molecules: Expression. Assembly and Function, edited by: Urban and Chicz, 1996, R.G. Landes Company, Austin, TX). Protein degradation by the proteasome generally results in oligopeptides of about seven to nine amino acids in length, but can vary from about three to about 30 amino acids in length (Baumeister *et al*., 1998, *Cell.* 92:367-380). In the ER, these peptides bind to newly synthesized MHC class I receptors which are transported to the plasma membrane and presented at the cell surface.

In the MHC class II pathway of antigen presentation, a protein or organism or foreign object is first endocytosed or phagocytosed, and is subsequently degraded into peptides of various lengths by endosomal or lysosomal enzymes such as cathepsins. Endogenous proteins which are found in endosomal-like vesicles are also processed into peptide fragments. In fact, these represent the majority of class II ligands. Stable degradation intermediates (peptides) are loaded onto MHC class II receptors, promoted by the MHC class II peptide loading facilitator HLA-DM (Roche, 1995, *Immunology* 3:259-262; Germain *et al.,* 1993, *Ann. Rev. Immunol.* 11:403-450; Tulp *et al.,* 1994, *Nature* 369:120-126). Thus, MHC class I and MHC class II receptors appear to provide a universal tool for the cataloguing, profiling, and characterizing of most and potentially all of the proteins present in a given cell.

For purposes of clarity, the following description refers mostly to the use of MHC class I and MHC class II receptors as tools for the practice of the invention. However, any other cellular multi-ligand binding receptor as defined and described above is intended to be within the scope of the invention. The skilled artisan would know how to practice the invention with the various different species of multi-ligand binding receptors as tools.

### Use of Cellular Multi-Ligand Binding Receptors as Tools to Catalogue, Profile and Characterize Ligands

As the skilled artisan will appreciate, for the practice of the instant invention, it is essential to isolate and purify the receptor/ligand complexes to a level of purity that allows for reproducible results, and in a manner such that the bound repertoire of ligands remains associated with the receptor during the process. Further, it is important subsequently to extract the bound repertoire of ligands at a level of specificity and efficiency that is sufficient for performing the subsequent characterization steps. Typically, the extraction process will be sufficiently efficient to recover each individual ligand at femtomole to picomole levels. A number of approaches may be taken to achieve these goals, and the skilled artisan will be able to identify and practice the methods and tools appropriate for such approaches and determine the stoichiometric amount of ligand purified from the quantified receptor preparation (Chicz *et al*., 1992, Nature 359:764-768; Chicz *et al.,* 1993, *J. Exp. Med.* 178:27-47; Chicz *et al.,* 1994, *Int. Immunol*. 6:1939-1649; Chicz and Urban, 1994, *Immunology Today* 15:155-160).

In the following, an example of practicing the invention with MHC class I receptors is described. The practice of the present invention is not contemplated to be limited to MHC receptors, but embraces the use of any multi-ligand binding receptor according to the above defined criteria. However, as MHC class I and class II receptors are known to bind a very complex repertoire of ligands, practice of the invention with MHC receptors may be the most challenging. Thus, with the guidance provided herein, the skilled artisan will be able to practice the invention with any other suitable multi-ligand binding receptor system. Of course, modifications of the very specific protocol described in the following will be required when the purification, extraction, and characterization processes are applied to other multi-ligand binding receptors. Moreover, for some multi-ligand binding receptors, additional considerations need to be taken into account. For example, some multi-ligand binding receptors, such as chaperones, chaperonins, and hsps, have ATPase binding domains, and bind the ligands in a stable manner only if ATP is bound to the domain, while hydrolysis of the ATP promotes release of the ligand (Kassenbrock and Kelly, 1989, *EMBO J*. 8:1461-1467; Blond-Elguindi *et al.,* 1993, *Cell* 75:717-728). In such cases, therefore, the purification of the receptor will be done in a manner such that the ATP remains stably bound to the ATPase binding domain, and the ligands may subsequently be released (e.g., by induction of ATP hydrolysis). *See Example 7.*

### Isolation and Characterization of EPTs Using MHC Receptors as Multi-Ligand Binding Receptors

General Considerations. The following method is a specific example of the immunoaffinity purification of class I HLA molecules followed by acid extraction of the EPT repertoire from the HLA molecules, reversed-phase HPLC partial fraction of the EPTs, and MALDI-TOF/MS analysis. As the invention is not limited to use of MHC receptors, it is likewise not intended to be limited to the specifically described protocols. As the skilled artisan will appreciate, numerous modifications are within the skill of the art. For example, various other protein purification, peptide separation and peptide analysis methods could be substituted for the specific methods described.

Class I HLA receptors are expressed on almost all nucleated cells and display their repertoire of non-covalently bound EPTs on the cell surface (Chicz and Urban, 1994, *Immunology Today* 15:155-159). Cell growth, harvest conditions and relative protein/ligand yield is determined experimentally depending on the cell line or tissue source in question. The skilled artisan will be able to determine the conditions for any particular cell line or tissue source desired for use. See, e.g., *Example 1.* For example, in a case where publicly available human B lymphoblastoid cell lines LG-2 (Chicz *et al.,* 1993, *JEM* 178:27-47), JY (Chicz *et al.,* 1993, *JEM* 178:27-47), and Priess (Chicz *et al*., 1993, *JEM* 178:27-47) have been used, 3-22 grams of each cell type may be re-suspended in 10 mM Tris-HCl, 1 mM dithiothreitol (DTT), 0.1 mM phenylmethylsulfonylflouride (PMSF), pH 8.0 at 4°C, and lysed in a homogenizer. The nuclei may be removed by sedimentation at 4,000x g for 5 minutes and the pellets washed and re-pelleted until the supernatants are clear. All the supernatants may be pooled and the membrane fraction harvested by sedimentation at 175,000x g for 40 minutes. The pellets may then be re-suspended in 10 mM Tris-HCl, 1 mM DTT, 1 mM PMSF, 1-4% Nonidet P-40 (NP-40). The unsolubilized membrane material may be removed by sedimentation at 175,000x g for 2 hours, and the NP-40-soluble supernatant fraction used for subsequent receptor-ligand purification.

Historically, preparative immunoaffinity purification of membrane bound glycoproteins have utilized soft gel polysaccharides (cellulose, agarose, and cross-linked dextrans) as the chromatographic media. However, these supports have limited mechanical strength, precluding the use of high flow rates, and their average particle size has the effect of decreasing resolution and increasing separation time. Modernizing this protocol by incorporating in-line, high-performance liquid-chromatography (HPLC) separations throughout the purification scheme improves the protein yield, reduces the number of manipulations, and eliminates the exposure of receptor-ligand complexes to extensive dialysis. Furthermore, by automating the purification system, the time required to purify protein/ligand complexes can be lowered from about 7 to 8 days down to a matter of about 3 to 4 hours per HLA molecule. This reduction in time is important because although protein/ligand complexes are quite stable, the interaction is not covalent and peptides can be released over time. In addition, this strategy can be conveniently coupled to use of other chromatographic supports including microcapillary reversed-phase chromatography (RPC) for the separation of extracted EPTs, followed by mass-spectrometry analyses. For example, for the purpose of the invention, protein/ligand purification based on the immunoaffinity chromatography method of Gorga *et al*., 1987, *J*. *Biol. Chem.* 262:16087-16094, may be modified to withstand the increased back pressure associated with mechanically produced high mobile phase flow rates from high-pressure liquid chromatography (HPLC) instruments.

In a preferred embodiment of the invention, a system referred to herein as the "Trident" system is used for the isolation and characterization of EPTs. The Trident system is an automated, in-line protein/peptide purification and analysis system. This system can be divided into three parts. Trident I encompasses the purification of protein/ligand complexes directly from the solubilized membrane preparation of a cellular lysate. Trident II focuses on the EPT extraction and separation components. Finally, Trident III achieves both EPT mass analysis and sequence identification. The skilled artisan will know how to optimize the instrumentation of each phase of the Trident system to optimize the time and effort required to identify EPTs derived from tissue-specific expressed proteins, for any given multi-ligand binding receptor.

### Trident I: Immunopurification of HLA Class I Receptors as Examples of a Multi-Ligand Binding Receptor

A number of important specifications have been introduced into Trident I. Dual-piston variable speed 10 *µ*l stroke volume high pressure pumps (10 *µ*l/min to 9.99 ml/min flow rate range) have been employed to achieve a dynamic range capable of generating both high resolution protein and peptide separations. This allows Trident to perform all the protein immunoaffinity chromatography methods (flow rates ranging from 0.25-9.99 ml/min) as well as microbore and microcapillary reversed-phase chromatography (RPC) separations of peptides at flow rates between 3 and 50 *µ*l/min in-line with continuous flow of mobile phase. Next, multiple 10-port high pressure switching valves are utilized to allow appropriate flow paths for automated column loading and serial elution of up to five individual mAb-specific immunoaffinity columns. These modifications empower a single HPLC unit to automatically purify up to five allotype-specific HLA molecules from a single lysate preparation without manipulation of the effluents or reloading of collected fractions. Two 7-port high pressure switching valves can be added to increase the number of individual columns to be eluted.

Multi-modal protein purification using HPLC columns is achieved by coupling the chromatographic procedures in series with automated switching valves, which direct the protein/ligand containing effluent to subsequent columns in the sequence. Each column effluent can be monitored at multiple UV wavelengths, pressure, and pH. High strength, large throughpore perfusion sorbents (polystyrene; 6000-8000 Å throughpores and 500-1000 Å diffusive pores, 50 *µ*m) coated and crosslinked with a hydrophilic stationary phase to which Protein A is covalently attached (POROS A^{™}; Perseptive Biosystems, Framingham, MA) can be utilized to allow for fast flowrates (up to 20 ml/min). The desired HLA-specific mAb can be attached to the POROS A^{™} resin as follows: Purified mAb is first dialyzed into 100 mM borate buffer pH 8.2 and then concentrated to >10 mg/ml. POROS A^{™} resin (PerSeptive Biosystems) is prepared for coupling by washing with 10 column volumes of 100 mM borate buffer pH 8.2. The supernatant is removed and the mAb solution added to the resin and mixed for 30-45 minutes. Ten column volumes of freshly prepared crosslinker (40 mM dimethyl pimelimidate/200 mM triethanolamine, pH 8.2) are then added to the resin and allowed to react at room temperature for 35-45 minutes. Afterwards, the resin is sedimented and the supernatant removed. To quench any remaining crosslinker, the resin is next suspended in 10 column volumes of 20 mM ethanolamine, pH 8.2, for 10 minutes (this step is repeated two times). At this stage, the resin can be packed into the column hardware and any non-crosslinked mAb removed by low- pH washes. Once characterized, the immunoaffinity columns are ready for use.

After the solubilized membrane preparation is loaded onto the columns, the columns are extensively washed using 50 column volumes of 20 mM MOPS/140 mM NaCl/0.1% DOC/0.05% NaN₃ at pH 8.0, followed by 100 column volumes of 10 mM Tris/0.1% DOC/0.05% NaN₃ at pH 8.0. Next, the protein-ligand complex is eluted from the immunoaffinity support using 3.5 column volumes of 50 mM carbonate/0.1% DOC/0.05% NaN₃ at pH 11.5.

The perfusion sorbents ideally have large throughpores which allow high velocity flowrates and also facilitate the cleaning/recycling of columns after protein/lipid fouling. Using this system allows reproducible chromatographic analyses and the purification of protein/ligand complexes from a specific immunoaffinity column in about three to four hours.

In Trident I, the solubilized membrane preparation described above is pumped through pre-clearing columns (chromatographic matrix and normal mouse serum-matrix) before the protein/ligand-containing effluent is directed towards a single (or series of) specific immunoaffinity column(s) using 50 column volumes of 10 mM Tris/0.1% NP-40/0.05% NaN₃ at pH 7.8. The immunoaffinity columns are then extensively washed using 50 column volumes of 20 mM MOPS/140 mM NaCl/0.1% DOC/0.05% NaN₃ at pH 8.0, followed by 100 column volumes of 10 mM Tris/0.1% DOC/0.05% NaN₃ at pH 8.0. Next, the protein/ligand complex is eluted from the immunoaffinity support using 3.5 column volumes of 50 mM carbonate/0.1% DOC/0.05% NaN₃ at pH 11.5.

The yields for total class I protein from a given cell line will vary. The average number of HLA class I molecules expressed on the surface of a given cell varies from 2 x 10⁴ to 5 x 10⁴ for non-professional antigen presenting cells, to 7 x 10⁴ to 7 x 10⁵ for professional antigen presenting cells (e.g., B-cells and macrophages). Table I (below) provides experimentally determined yields accomplished using the Trident system as well as those achieved using conventional chromatography for several cell lines (see reference sources).

**TABLE I**

| **Cell Line** | **Number of Cells Used** | **Harvested Weight of Cells** | **HLA-A (*µ*g/g cells)** | **HLA-B/-C (*µ*g/g cells)** | **Total Class I (*µ*g/g cells)** | **Reference** |
|---|---|---|---|---|---|---|
| JY | | 15 g | 16 | 47 | 63 | 1 |
| JY | | 20 g | 16 | 47 | 63 | 1 |
| JY | | 22 g | 16 | N/D | N/D | 1 |
| JY | | 18 g | 44 | 67 | 111 | 1 |
| JY | 10¹⁰ | | 19-31 | | | 2 |
| 9052 | 10¹⁰ | 10 g | | | 130 | 3 |
| LG-2 | | 200 g | | | 50 | 4 |
| LG-2 | | 10 g | 25 | 60 | 85 | 1 |
| LG-2 | | 100 g | | 12 | | 5 |
| U937 | 10¹⁰ | | | | 21 | 3 |
| U937 | 10¹⁰ | | | | 18 | 3 |
| HeLa S3 | 10¹⁰ | | | | 25 | 1 |
| HeLa S3 | 10¹⁰ | | | | 20 | 1 |

| | | | | | | |
|---|---|---|---|---|---|---|
| ¹ as disclosed herein ² Tsomides *et al.,* 1991, *Proc. Natl. Acad. Sci. USA* 88:11276 ³ Harris *et al.,* 1994, *Tissue Antig.* 44:65 ⁴ Gorga *et al.,* 1986, *J. Biol. Chem.* 262:16087-1694 ⁵ Urban *et al.,* 1994, *Proc. Natl. Acad. Sci. USA* 91:1534-1538 | | | | | | |

The yields of EPTs will vary not only with the number of multi-ligand binding receptors expressed per cell, but also with the rate of protein turnover in a given cell, tissue or organ type. If the level of protein turnover is high, and a cell has a high level of protein synthesis, the number of EPTs can be expected to be higher. In the case of HLAs, the normal repertoire of HLA associated peptides has an occupancy level of 0.1 - 1% for any given peptide, based on a 1:1 stoichiometry of EPT and HLA receptor. Thus, the yield of EPTs from HLA receptors will be an experimentally determined value based on the expression level of the full length EPT source protein and the number of HLA receptors obtained from the target cell line.

### Trident II: Isolation and Separation of the EPT Repertoire

Isolation and separation of the cell's repertoire of EPTs is accomplished in Trident phase II. After alkaline elution of the HLA/EPT complexes from the immunoaffinity supports, the HLA-bound EPT repertoire is extracted from the complexes by solid-phase extraction through a series of multi-modal chromatography sorbents. An anion-exchange chromatography (AEC) support (POROS 20 HQ/M^{™} (PerSeptive Biosystems, Framingham, MA), 6000-8000 Å throughpores and 500-1000 Å diffusive pores, 15-25 *µ*m) is employed as the first sorbent in the Trident II solid-phase extraction protocol. The AEC column functions to capture the intact protein/ligand complex as it elutes off the immunoaffinity column. Next, the AEC column is washed, for example, with 20 column volumes of 50 mM carbonate at pH 11.5, to remove the detergent component of the immunoaffinity mobile phase eluent. One column volume of 10% TFA/H₂O and an increase in temperature to 70°C is next applied to the AEC column to protonate the adsorbed protein/ligand complex and elute off the bound EPT repertoire. Due to the relatively high acidic charge distribution on the surface of the HLA protein, the acidic conditions do not affect the electrostatic interactions between the protein and the charged AEC column. Thus, only the peptide ligands are allowed to pass through the column, while the now denatured proteins remain adsorbed to the AEC support. The effluent from the AEC column is directed onto a polymeric polystyrene crosslinked divinylbenzene reversed-phase chromatography (RPC) column (POROS R2/H^{™}, 6000-8000 Å throughpores and 500-1000 Å diffusive pores, 8-10 *µ*m), which acts as a peptide capture column (PCC). Once the EPT repertoire is adsorbed onto the PCC support, mobile phase exchange is accomplished with, e.g., a 20 column volume wash using 0.1% TFA/1% acetonitrile/H₂O. EPT isolation is complete at this stage. A second reversed-phase separation is next utilized to fractionate the isolated EPT repertoire. The individual peptide ligands are separated based on relative hydrophobicity using a second RPC column, a silica based C₁₈ support (300 Å, 5 *µ*m; Vydac, Hesperia, California). The EPT repertoire is eluted from the PCC support using a non-linear gradient of buffer A/buffer B at a constant flow rate of 5-50 µl/min depending on the RPC column dimensions:
0-63 minutes 5%-33% buffer B; 63-95 minutes 33%-60% buffer B; 95-105 minutes 60%-80% buffer B; where buffer A is 0.06% TFA/5% acetonitrile/H₂O and buffer B is 0.055% TFA/5% H₂O/acetonitrile. The chromatographic analysis is monitored by UV absorbance at multiple wavelengths (210, 254, 277, 292 nm) to identify peptide bonds as well as EPTs containing conjugated delocalized π-electrons (aromatic amino acids). The more hydrophobic individual ligands elute later in the gradient with increasing percentage of organic modifier. The flow stream is interfaced with a 50:1 micro-fraction MALDI-TOF/MS sample plate collector split to allow simultaneous sample collection and MALDI-TOF/MS sample preparation. In this manner, 2% of the collected sample is immediately prepared for mass analysis (Trident III), while the remaining 98% of each separated EPT fraction is collected and stored for future screening. The output of Trident II is a collection of fractions, each containing multiple EPTs, with fraction separation based on relative hydrophobicity, a function of amino acid composition and sequence.

As an alternate approach to the solid-phase extraction described above, a batch mode acid extraction can be used to isolate EPTs from purified HLA molecules. In this procedure, the solution containing the purified detergent-soluble protein/ligand complexes is first buffer exchanged and concentrated into a low volume (about 1/15 to 1/30 original volume) and more neutral pH mobile phase, *e.g.,* 20 mM MOPS, 140 mM NaCl, 0.1% DOC, at pH 8.0. (E.g., where the collected sample volume is 10-15 ml, it is first concentrated to 0.5-1 ml, then down to 50-100 *µ*l with an ultra-filtration device.) Following dilution to 1 ml with 10% acetic acid, the solution containing the complexes is heated to 70°C for 15 minutes, thereby dissociating the EPTs from the HLA molecules. The EPT repertoire is then separated from the now empty HLA heavy and light chains by size exclusion (differences in Stokes radius), using ultrafiltration devices with a 3-10 kDa molecular weight cutoff. The solution containing the mixture of EPTs can then be loaded onto the RPC column for fractionation as described above.

### Trident III: Mass and Sequence Analysis of Isolated EPTs

The final stage of Trident specifically addresses the mass and sequence analysis of isolated EPT mixtures. The most critical step in the analysis of proteins and peptides by mass spectrometry is an acceptable method of rendering charged molecular species (ionization). Advances in sample ionization processes have propelled mass spectrometry from a peripheral technique to a central component of protein and peptide characterization. Specifically, new developments in electrospray-ionization (ESI-MS) and matrix-assisted laser desorption ionization time-of-flight mass spectrometry (MALDI-TOF/MS) now provide consistent and routine mass and sequence analyses. Advances in MALDI-TOF/MS have made this technology an especially attractive analytical tool for the mass and sequence analysis of complex mixtures of low abundance peptides. Four prominent features of MALDI-TOF/MS make this approach superior for the analysis of EPTs. First, MALDI-TOF/MS spectra tend to be less complicated than those collected using electrospray ionization mass spectrometry (ESI/MS) because the ionization process favors the formation of single (1+) ions rather than multiply charged ions (1+, 2+, 3+, etc.). This is an important consideration when comparing spectra of multi-component samples. Second, this technique uses minimal amounts of sample: sub-femtomole amounts for mass analyses and sub-picomole amounts for sequence analyses. Third, the high mass accuracy and superior mass resolution afforded using this technique are not achievable using most alternative mass spectrometry techniques. Finally, primary sequence information can be generated using two complementary modes of daughter ion fragmentation.

Superior mass accuracy and resolution data are critical to properly screen fractions for EPT analysis. The fractions are first screened for complexity and relative abundance using the low resolution linear mode of MALDI-TOF/MS analysis. The collected spectra provide an accurate estimate of the number of individual peptides present and the relative ionization of each. Because each fraction from the primary RPC separation can contain as many as 50-150 individual EPTs, high resolution combined with high mass accuracy is currently the most reliable method to screen the fractions for complete peptide characterization (Vestal *et al*., 1995, *Rapid Communication in Mass Spectrometry* 9:1044-1050). For example, techniques with lower resolving power (at the current state of art), i.e., ion trap or triple quadruple mass spectrometers equipped with electrospray ionization sources, and which have a normal resolution of -1,000-2,000 in the m/z=1,000-2,000 range at femtomole sensitivity in full scan mode, are currently less reliable for characterizing peptides with mass differences of 1-3 daltons or less. The difficulty is mostly due to the inability of these alternative techniques to properly resolve the isotopic distribution of a single peptide. Of course, such techniques may be improved in the course of technical development, and as a result be better suited for the purposes of the invention.

MALDI-TOF/MS instruments equipped with extended flight paths and delayed extraction ionization fields can achieve superior mass accuracy and resolution. The exceptional performance of this instrumentation enables the reliable collection of multi component spectrum while permitting the mathematical subtraction of one spectrum from another. Moreover, the high resolution and mass accuracy allows for more accurate determination of the total number of individual masses in a given sample fraction. Coupled with the highly reproducible chromatographic separations achieved with Trident phases I and II, EPT analysis of samples isolated from different sources of interest, e.g., from disease and non-disease linked tissues, different organ or tissue types, different developmental or metabolic stages of a cell, tissue or organ, etc., becomes possible by using a subtraction algorithm to identify the novel ligands derived from either unique or even mutated source proteins expressed in the disease linked tissue. The individual EPT masses from the normal cell can be subtracted from the EPT repertoire of the disease related cell leaving only those EPTs that are associated with either novel or mutated proteins. Once identified as novel EPT targets, these EPTs are then sequenced for complete identification, *see, infra.*

Another advantage of the use of MALDI-TOF/MS (as of the current state of art) relates to its ability to generate structural information for sequence determination of biomolecules. Fragment ions can be generated in MALDI-TOF/MS by a phenomenon described as post-source decay (PSD). Briefly, the sample analyte ions undergo "delayed" fragmentation/neutralization reactions during flight stemming from multiple collisions with matrix molecules during gas phase plume expansion and ion acceleration. MALDI-TOF/MS is unique in forming pre-excited precursor ions which move at a fairly high kinetic energy over a long distance where they can undergo uni-molecular decomposition with or without further collisional activation. Using PSD analysis, complete sequence information can be generated from the daughter ion fragmentation patterns. The fragmentation patterns are different from those observed using high energy four-sector instruments or other tandem mass spectrometers such as electrospray triple-quadruple instruments. Furthermore, MALDI-TOF/MS sensitivity is at least two orders of magnitude better than the aforementioned mass spectrometry approaches due to the high overall yield of fragment ions and the high ion transmission inherent in TOF instruments. However, to enhance PSD analysis even further, a collision cell can be introduced to the system. With a collision cell in place, high energy collision induced dissociation (CID) spectra can be collected, which produce complementary fragmentation patterns as compared to PSD spectra. The combined data sets produce additional structural information for the sequence determination of unknown peptides.

A complementary technique to MALDI-TOF/MS for the sequence analysis of low femtomole amounts of peptide is ion-trap mass spectrometry. First, the mass range of ion-trap instruments has recently been extended to include linear mass calibration and ion fragmentation for peptides. With these advances in place, several commercial ion-trap instruments are now available. Briefly, the strength of the ion-trap technology is the capability to isolate a given ion while ejecting all the non-selected ions from the instrument, hence the name ion-trap. This is accomplished through the use of non-linear multiple fields, advanced resonance frequency electronics, and optimized ring and endcap designs in the trap, which enhance the ion ejection speed and extend the useful mass range of the instrument. The end result is the ability to perform multiple fragmentation experiments on a given ion (known as MS⁽ⁿ⁾), which extends the amount of information collected from peptide fragmentation. This technology also allows the continuous flow of sample into the trap, with only the target ion being retained to a degree necessary for efficient fragmentation of the target ligand. In this manner, low abundance sample can be concentrated within the instrument to perform the sequence experiment. Sequencing is manifested by performing a ZoomScan or limited mass range scan on a known mass. In this mode, the instrument can operate at high sensitivity and resolution, but at the cost of scanning only a limited mass range. The decreased sensitivity and resolution compromises the detection of most ions in complex mixtures. For these reasons, the combination of MALDI-TOF/MS with ion-trap MS may lead to faster sequence identification of EPTs.

Mass spectra collected using reflector MALDI-TOF/MS analysis normally have a mass accuracy near 0.01% using external calibration, and can achieve mass accuracy within 10-50 ppm using internal calibration. This is sufficient for use in mass matching protocols, where theoretical mass values of peptides are compared to a linear sequence from a target protein. Novel mass values obtained by the subtractive algorithm are used to search out all possible mass matches within the amino acid sequence of the target protein. Post-translational modifications can be taken into consideration during these analyses. Those prospective peptide masses matching potential strings within the target protein (within a tolerance of 0.01% using monoisotopic mass values) are further analyzed. Mass matching is useful because it focuses the ensuing analysis on sequence verification as opposed to complete unknown sequence determination. Because the mass matching protocol described above matches the linear peptide sequence with the experimentally reported mass value, the fragmentation patterns, including all ion types (b, y, a, d, w series), immonium series, and deamidated and dehydrated forms can be mathematically predicted. Thus, peptide masses chosen by mass matching can be sequenced and the experimentally determined PSD and CID spectra (collected by either MALDI-TOF/MS or ion-trap MS) are compared to the theoretical predicted spectra to verify the mass matching by sequence analysis. Once a candidate peptide has been properly identified, one may produce, as a control, synthetic peptide analogues and collect HPLC retention analyses, mass analyses, and most importantly PSD and CID fragmentation patterns to compare them to those used originally to determine the sequence, to confirm the unknown sample determination.

Using the methods described above, the sequences of EPTs from both novel proteins and proteins already represented by sequence data in public databases can be determined. The data profiles that are compiled for each sample are displayed in multi-dimensional space. Typically, each peptide has a profile that is at least two dimensional, with a first dimensional coordinate representing its mass, and the second coordinate representing the time of elution, i.e., fractionation. Depending on the separation methods chosen, the position of a ligand on the fractionation coordinate may correspond to its relative hydrophobicity (*i.e*., % of eluting buffer, *e.g*., acetonitrile or isopropanol, required for elution), its charge (measured by ion exchange, *i.e*., relative concentration of salt, *e.g*., NaCl, required for elution; *e.g*., AEC fractionates according to negative charge and CEC fractionates according to positive charge), its hydrophilicity (measured by normal phase chromatography), its hydrophobicity and H₂O hydration (measured by hydrophobic-interaction chromatography), its affinity for metal chelate ligands such as Cu⁺², Ni⁺² and Fe⁺³ (measured by immobilized metal affinity chromatography, or IMAC) or its mobility (measured by capillary electrophoresis, i.e., time for a peptide to come out of capillary based on electrical field). See, Alpert, 1988, *J*. *of Chromatography* 444:269-274; Crimmins et *al.,* 1988, *J. of Chromatography* 443:63-71; Dizdaroglu, 1982, J. *of Chromatography* 237:417-428; Nakawaga et *al.,* 1988, *Analytical Biochemistry* 168:75-81; Alpert, 1990, *J. of Chromatography* 499:177-196; Tomlinson *et al.,* 1997, *J. Am. Soc. Mass Spectrom.* 8:15-24; Tomlinson *et al.,* 1996, *J. of Chromatography* 744:273-278; Colovai *et al.,* 1994, *Tissue Antigens* 44:65-72; and Tsomides et *al.,* 1991, *Proc. Natl. Acad. Sci. USA* 88:11276-11280. Each ligand can be further characterized by a third coordinate representing its intensity of ionization (corresponding to its individual amino acid sequence in the case of an EPT ligand).

In other embodiments, the ligand may be characterized in still further dimensions, e.g., by determining more than one of a ligand's (or pool of ligands') separation parameters. For example, one coordinate may represent a ligand's mobility, as determined by capillary electrophoresis, and another coordinate may represent a ligand's hydrophobicity, as determined, e.g., by reversed HPLC. A coordinate may be added, or may replace one of the above, representing a ligand's charge, as determined by, e.g., ion exchange chromatography (*e.g.,* AEC according to negative charge and CEC according to positive charge). Another coordinate may be added, or may replace any of the above, representing a ligand's hydrophilicity, as determined, e.g., by normal-phase chromatography. Another coordinate may be added, or may replace any of the above, representing a ligand's hydrophobicity and H₂O hydration, as determined, e.g., by hydrophobic-interaction chromatography. Yet another coordinate may be added, or may replace any of the above, representing a ligand's modifications, such as acetylation or heavy H₂O content. The skilled artisan will be able to determine any other parameters that could be added to or replaced by any of the above, to characterize a ligand's or plurality of ligands' profile.

The sensitivity of mass spectrometer-based analysis of EPTs is dependent on the individual sample (with respect to ionization), but currently falls in the range from about 10⁻¹⁶ to about 10⁻¹⁵ moles for simple mass analysis and from about 10⁻¹⁵ to about 50 x 10⁻¹⁵ moles for sequence identification. Thus, as the skilled artisan will appreciate, enough sample must be provided for this type of analysis to provide meaningful information.

### Amplification of the Number of Multi-Liqand Binding Receptors Expressed by the Cells of Interest

In some cases, it may be desired to take measures to amplify the number of multi-ligand binding receptors prior to their isolation. Amplification protocols include, but are not limited to (a) engineering of recombinant soluble multi-ligand binding receptors into the cell line of interest; (b) a cell fusion approach for immortalizing primary cells by fusing them to immortalized cell lines, e.g., primary cells expressing a particular set of multi-ligand binding receptors, are fused to tumor cells engineered to express soluble multi-ligand binding receptors; (c) introducing immortalizing vectors into the cell of interest; (d) feeding the cells with substances that increase expression of a particular multi-ligand binding receptor; or (e) growing cells in athymic or SCID mice (*e.g*., in the case of tumor cells or other primary cells that do not grow *in vitro*).

As to (a), recombinant vectors designed to drive the expression of one or several multi-ligand binding receptors may be generated by methods generally known in the art. Briefly, DNA cloning is used to construct an expression vector containing the coding sequence of a particular multi-ligand binding receptor and appropriate transcriptional/translational control elements. These methods include in vitro recombinant DNA techniques, synthetic techniques, and <i>in vivo </i> recombination/genetic recombination. See, for example, the techniques described in Sambrook *et al*., *supra*; and Ausubel *et al*., Current Protocols in Molecular Biology, Greene Publishing Associates and Wiley Interscience, N.Y. (current edition). The vector can be a virus or a plasmid.

In cases where the cells of interest can proliferate in culture, as is true for, e.g., kidney, liver, lung, thymus, intestine, colon, neural cells, mesenchymal cells, stem cells, etc., the recombinant DNA may be introduced into the cells *in vitro.* Numerous techniques are known in the art to introduce and express, stably or transiently, recombinant DNA *in vitro,* i.e., in cultured cells. See, Sambrook et *al.,* 1989, *supra;* Ausubel *et al*., *supra.* In cases where the cells of interest cannot be grown in culture, methods and tools have to be chosen that allow introduction of the recombinant DNA. For example, in mammalian cells, a number of viral based expression systems, e.g., packaged into intact virus particles, may be utilized. In cases where an adenovirus is used as an expression vector, the multi-ligand binding receptor encoding sequence may be ligated to an adenovirus transcription/translation control complex, e.g., the late promoter and tripartite leader sequence. This chimeric gene may then be inserted in the adenovirus genome by *in vitro* or *in vivo* recombination. Insertion in a non-essential region of the viral genome (*e.g.,* region E1 or E3) will result in a recombinant virus that is viable and capable of expressing the gene encoding the receptor in infected hosts. *See,* for example, Logan and Shenk, 1984, *Proc. Natl. Acad. Sci. USA* 81:3655-3659. Alternatively, the vaccinia 7.5K promoter may be used. See, for example, Mackett *et al.,* 1982, *Proc. Natl. Acad. Sci. USA* 79:7415-7419; Mackett et *al.,* 1984, *J. Virol.* 49:857-864; Panicali *et al.,* 1982, *Proc. Natl. Acad. Sci. USA* 79:4927-4931. Other suitable viral systems include, but are not limited to, SV40 based viral systems, pox based viral systems, EBV based viral systems, lentiviral systems, HSV based viral systems, and retroviral systems. *See, e.g.,* Kriegler, M., *Vectors, in*: Gene Transfer and Expression, ed. Kriegler, M. WH Freeman and Company, NY, 1990.

Suitable promoter systems for expression of the multi-ligand binding receptors include both constitutive promoters, viral promoters such as CMV, SV40, T7, adenovirus, and inducible promoters, such as the tet system, the glucocorticoid responsive element, the metallothionein promoter, interferon or prostaglandin receptor elements. Suitable promoter systems both for in vitro and for *in vivo* expression of the multi-ligand binding receptors in cells of interest can be found in Kriegler, M., *Vectors, in:* Gene Transfer and Expression, ed. Kriegler, M. WH Freeman and Company, NY, 1990.

As to (b), *supra*, the cells of interest may be fused with any type of immortalized cell expressing the appropriate multi-ligand binding receptor chosen for the particular experimental task using, for example, hybridoma techniques. See, Harlow and Lane, *supra*. For example, if introduction of MHC class I or MHC class II receptors into the cell of interest is desired, the cells may be fused to, *e.g.,* an immortalized B-cell line. The skilled artisan will be able to determine what immortalized cell line may be particularly useful for the introduction of a selected multi-ligand binding receptor into the cell of interest using techniques generally known in the art, but not limited to mRNA hybridization techniques using nucleic acid probes specific for various multi-ligand binding receptors, such as Northern blots, *in situ* hybridization, dot blots, RT-PCR, RNase mapping, or S1 nuclease mapping, or immunohistological techniques using antibodies specific for the multi-ligand receptor binding protein, such as Western blots, ELISA, FACS analysis, immunoprecipitation, or *in situ* immunostaining. See, among others, Sambrook *et al*., 1989 Molecular Cloning: A Laboratory Manual 2nd ed. Cold Spring Harbor Laboratory Press; Current Opinion in Molecular Biology, *supra*; Harlow and Lane, 1988, *supra.*

Furthermore, the cells of interest may be fused to immortalized cells that have been engineered to express a soluble multi-ligand binding receptor, such that the receptor is secreted from the fusion cell and can be conveniently collected and purified from the medium (in the case of cultured cells) or body or tissue fluid (where the fused cells are implanted in a host). Suitable methods for generating such recombinant immortalized cells can be found in Sambrook *et al*., 1989, *supra*; Ausubel *et al*., *supra*. Methods for fusing cells can be found in Harlow and Lane, 1988, *supra.*

As to (c), *supra*, suitable immortalizing vectors include, but are not limited to, EBV virus-based vectors (preferably if the objective is to transform B-cells), SV40-based vectors, polyoma large T antigen based vectors, BPV, CMV based vectors, and any other vector containing suitable viral or retroviral elements. Furthermore, the cells may be immortalized by retroviral infection or infection with other virus types, typically when virus is used at an MOI where most cells are transduced. A general review of suitable immortalizing vectors is found in Kriegler, M., *Vectors, in:* Gene Transfer and Expression, ed. Kriegler, M. WH Freeman and Company, NY, 1990.

As to (d), expression of certain multi-ligand binding receptors may be upregulated by contacting the cells with, e.g., cytokines. For example, expression of HLA may be upregulated by contacting the cells with γ-interferon.

As to (e), many tumor cell lines that do not grow *in vitro* do grow in immunocompromised mice, such as SCID or nude mice. Methods for growing tumor cells in such mice are well established in the art. Bumpers *et al*., 1994, *J. Clin. Invest.* 94:2153-2157; Bumpers *et al.,* 1996, *J. Surg. Res.* 96:282-288; WO 97/8300-A2.

### Generation of Profiles Representing Ligands Extracted From a Multi-Ligand Binding Receptor of a Cell of Interest

In one embodiment, the invention provides profiles representing a plurality of ligands which have been extracted from at least one preselected multi-ligand binding receptor of a cell of interest. The invention further provides procedures and tools for generating such profiles.

Generally, the profiles of the invention may represent ligands extracted from any multi-ligand binding receptor within the scope of the invention. Preferably, the ligands are peptides or proteins. Generally the profile may represent ligands extracted from preselected multi-ligand binding receptor(s) isolated from any type of cell of interest. In one embodiment, the profile represents a plurality of ligands which have been extracted from a preselected multi-ligand binding receptor of a cell of interest that is not a professional antigen presenting cell. In an alternative embodiment, the ligands are extracted from a preselected multi-ligand binding receptor of a cell of interest that is not a B-cell. In another embodiment, the ligands are extracted from a preselected multi-ligand binding receptor of a cell of interest that is not a macrophage. In yet another embodiment, the ligands have been extracted from a preselected multi-ligand binding receptor of a cell of interest that is a professional antigen presenting cell, i.e., a B cell, macrophage, or dendritic cell. In yet another embodiment, the profile comprises a representation of each of a plurality of defined ligands which have been extracted from at least two preselected multi-ligand binding receptors of a cell of interest.

In preferred embodiments of the invention, the ligand is a protein, or even more preferably a peptide. Typically, such peptide or protein ligands are derived from proteins expressed within the cell, and thus reflect a subset of the proteins expressed within the cell. Generally, the profile represents peptide or protein ligands extracted from one multi-ligand binding receptor and having at least ten distinct core peptides, as defined above. If the multi-ligand binding receptor is an MHC class I or an MHC class II receptor, and the ligands represented in the profile have been extracted form a single allotype, the profile represents at least 40 (e.g., at least 50) ligands having distinct core peptides. More preferably, the profile represents at least 70 ligands having distinct core peptides: for example, at least 100, at least 200, or most preferably at least 500. If the profile includes a representation of at least 70 ligands having distinct core peptides, such ligands may be extracted from one or more different multi-ligand binding receptors.

The total number of distinct ligands represented by the profile is typically at least 50, preferably at least 500, more preferably at least 1000, and most preferably at least 2,000 through 10,000. These numbers include peptide or protein members with or without overlapping amino acid sequence, i.e., which may not have distinct core peptides.

The ligands represented in the profile may represent at least 10% of the proteins expressed in the cell of interest, for example at least 20%, 50% or even 80% As the skilled artisan will appreciate, the complexity of the profile will largely depend on the multi-ligand binding receptor(s) and/or the particular cell type chosen for the production of the profile.

In preferred embodiments, the multi-ligand binding receptor is an MHC class I or an MHC class II receptor. In alternative embodiments, the multi-ligand binding receptor is a chaperone, e.g., calnexin, calreticulin, BIP, grp96, and/or grp94. In alternative embodiments, the multi-ligand binding receptor is a chaperonin, or an hsp, *e.g*., hsp60, hsp65, hsp70, hsp90, and hsp25. Alternatively, the multi-ligand binding receptor is a proteasome complex or a binding component thereof, or another component of the ubiquitin pathway, e.g., an E2 ubiquitin carrier protein (e.g., CDC34), an E3 ubiquitin ligase (e.g., cyclosome or components thereof, G1/SKP1/Cullin/F-box complex, E3α, hectdomain protein), an unfoldase, or an hsp100. Other possibilities are a mannosidase, a N-glycanase, the mannose receptor, or a trafficking or retention protein, e.g., the KDEL receptor. Profiles, of course, may be generated by extracting ligands from any possible combination of a plurality of the multi-ligand binding receptors within the scope of the invention.

In most preferred embodiments, the multi-ligand binding receptor is an allelic variant of an MHC receptor, e.g., an H-2 receptor, or an HLA receptor, such as a HLA class II receptor, e.g., HLA-DR, HLA-DQ, or HLA-DP, or an HLA class I receptor, e.g., HLA-A, HLA-B, HLA-C, HLA-E, HLA-F, or HLA-G receptor, or a combination of two or more of them. In one specific embodiment, the profile consists of representations of ligands extracted from an HLA-A allotype, but not an A-0101, A-0201, A-0202, A-0203, A-0204, A-0205, A-0206, A-0207, A-0214, A-0301, A-0302, A-1101, A-2402, A-2601, A-2901, A-3101, A-3201, A-3302, A-6801, or A-6901. In another specific embodiment, the profile consists of representations of ligands extracted from an HLA-A allotype, but not an A-0101, A-0201, A-0204, A-0205, A-0206, A-0207, A-0214, A-0301, A-1101, A-2402, A-2901, A-3101, A-3302, A-6801, or A-6901.

In another specific embodiment, the profile consists of representations of ligands extracted from an HLA-B allotype, but not a B-0702, B-0801, B-1401, B-1402, B-1501, B-1502, B-1508, B-1509, B-1513, B-1516, B-1517, B-1801, B-2701, B-2702, B-2703, B-2704, B-2705, B-2706, B-3501, B-3503, B-3701, B-3801, B-39011, B-3902, B-4001, B-40012, B-4006, B-4401, B-4402, B-4403, B-4601, B-5101, B-5102, B-5103, B-5201, B-5301, B-5401, B-5501, B-5502, B-5601, B-5701, B-5702, B-5801, B-5802, B-6701, B-7301, or B-7801. In another specific embodiment, the profile consists of representations of ligands extracted from an HLA-B allotype, but not a B-0702, B-0703, B-0705, B-0801, B-1402, B-1501, B-1502, B-1508, B-1509, B-1513, B-1516, B-1517, B-1801, B-2701, B-2702, B-2703, B-2704, B-2705, B-2706, B-3501, B-3503, B-3701, B-3801, B-39011, B-3902, B-4001, B-40012, B-4006, B-4402, B-4403, B-4601, B-5105, B-5102, B-5103, B-5201, B-5301, B-5401, B-5501, B-5601, B-5701, B-5702, B-5801, B-5802, B-6701, B-7301, or B-7801.

In another specific embodiment, the profile consists of representations of ligands extracted from an HLA-C allotype, but not a C-0101, C-0102, C-0301, C-0304, C-0401, C-0602, C-0702, or C-1601.

In another specific embodiment, the profile consists of representations of ligands extracted from an HLA-E allotype, but not an E-101.

In another specific embodiment, the profile consists of representations of ligands extracted from an HLA-G allotype, but not a G-01012.

In another specific embodiment, the profile consists of representations of ligands extracted from an HLA-DR allotype, but not a DR-B1*0101, DR-B1*1501, DR-B1*1502, DR-B1*1503, DR-B5*0101, DR-B5*0201, DR-B1*0301, DR-B1*1601, DR-B1*0401, DR-B1*0402, DR-B1*0403, DR-B1*0404, DR-B1*0405, DR-B1*0406, DR-B1*0408, DR-B1*0701, DR-B1*0801, DR-B1*09011, DR-B1*09012, DR-B1*1001, DR-B1*1001, DR-B1*1104, DR-B1*1111, DR-B1*1201, DR-B1*1301, DR-B1*1302, DR-B3*0101, DR-B3*0202, DR-B3*0301, or DR-B5*0101.

In another specific embodiment, the profile consists of representations of ligands extracted from a HLA-DR allotype, but not a DR-B1*0101, DR-B1*0102, DR-B1*0301, DR-B1*0401, DR-B1*0402, DR-B1*0404, DR-B1*0405, DR-B1*0407, DR-B1*0701, DR-B1*0801, DR-B1*09011, DR-B1*1101, DR-B1*1104, DR-B1*1201, DR-B1*1301, DR-B1*1302, DR-B1*1501, DR-B3*0202, DR-B3*0301, or DR-B5*0101.

In another specific embodiment, the profile consists of representations of ligands extracted from an HLA-DQ allotype, but not a DQ-A1*0101/B1*0501, DQ-A1*0102/B1*0502, DQ-A1*0201/B1*0201, DQ-A1*0501/B1*0201, DQ-A1*0301/B1*0401, DQ-A1*0401/B1*0402, DQ-A1*05012/B1*0301, DQ-A1*0102/B1*0602, DQ-A1*0301/B1*0301, DQ-A1*0301/B1*0302, or DQ-A1*0301/B1*0303.

In another specific embodiment, the profile consists of representations of ligands extracted from an HLA-DQ allotype, but not a DQ-A1*0101/B1*0501, a DQ-A1*0201/B1*0201, a DQ-A1*0301/B1*0301, a DQ-A1*0301/B1*0302, or a DQ-A1*0501/B1*0201.

In another specific embodiment, the profile consists of representations of ligands extracted from an HLA-DP allotype, but not a DP-A1*0102/B1*0201, DP-A1*/B1*0202, DP-A1*0101/B1*0301, DP-A1*0101/B1*0401, DP-A1*0201/B1*0401, DP-A1*0101/B1*0402, DP-A1*0201/B1*0902, or DP-A1*/B1*1401.

In another specific embodiment, the profile consists of representations of ligands extracted from an HLA-DP allotype, but not a DP-A1*0102/B1*0201, A1*0201/B1*0401, or A1*0101/B1*0301.

Furthermore, the invention provides methods for generating such profiles. Generally, such methods include the isolation of one or multiple types of multi-ligand binding receptors from a cell of interest under conditions that preserve association of the bound ligands, the subsequent extraction of the ligands bound to the receptor, and the characterization of the ligands according to selected chemical and physical parameters, such as the HPLC profiles (anion-exchange, cation-exchange, reversed-phase, normal phase, hydrophobic-interaction chromatography), capillary electrophoresis profiles (CE, AEC-CE, CZE, or CEC-CE), and mass spectrometry profiles (MALDI-TOF/MS, FTMS, ESI-TOF, MALDI-ITMS, ESI-Quadropole MS, ESI-Quadropole/TOF-MS, ESI-Sector MS, FAB-MS, or ESI-ITMS), or intensity of ionization, and the resulting properties. Depending on the method of ligand separation, a unique physical characterization may be derived. For example, reversed-phase chromatography separates individual peptides on the basis of their hydrophobicity. In this case the ligands are characterized according to their relative hydrophobicity. Ion-exchange chromatography differentiates on the basis of charge, i.e., AEC according to negative charge and CEC according to positive charge. Thus, in this case the ligands are characterized according to their relative charge. Normal-phase chromatography differentiates on the basis of relative hydrophilicity. In this case, therefore, the ligands will be characterized according to their relative hydrophilicity. Hydrophobic-interaction chromatography differentiates on the basis of hydrophobicity and H₂O hydration. Accordingly, the ligands are characterized based on their relative hydrophobicity and H₂O hydration. Capillary electrophoresis differentiates on the basis of charge depending on what polymeric coating is applied to the capillary. Thus, in this case the ligands are characterized according to their relative charge. Mass spectrometry methods (MALDI-TOF/MS, FTMS, ESI-TOF, MALDI-ITMS, ESI-Quadropole MS, ESI-Quadropole/TOF-MS, ESI-Sector MS, FAB-MS, or ESI-ITMSI) characterize the ligands according to their mass. Mass spectra of peptide fragmentation patterns are a way to determine a peptide's or protein's amino acid composition and/or sequence. Other methods of amino acid composition and/or sequence determination generally known in the art may be employed as well. Generally, the skilled artisan will know what ligand separation methods will be suitable and appropriate to characterize the ligands in a meaningful way and on the basis of selected chemical and physical parameters.

In one embodiment, the invention provides a method for the generation of a library or a profile comprising representations of at least 40 ligands (preferably at least 70, more preferably at least 100, and most preferably at least 500) having distinct chemical and/or physical characteristics In another embodiment, the method is for the generation of a profile representing a plurality of ligands which have been extracted from a preselected multi-ligand binding receptor of a cell of interest that is not a professional antigen presenting cell. In an alternative embodiment, the ligands are extracted from a preselected multi-ligand binding receptor of a cell of interest that is not derived from a B-cell or a macrophage. In again another embodiment, the method provides for the generation of a profile comprising representations of a plurality of defined ligands which have been extracted from at least two preselected multi-ligand binding receptors of a cell of interest.

In preferred embodiments of the invention, the ligand is a protein or a stable peptide intermediate of its biosynthesis or degradation. Typically, such peptide or protein ligands are derived from proteins expressed within the cell, and thus reflect a subset of the proteins expressed within the cell. Generally, the method provides for the generation of a profile representing multiple peptides, at least ten (and preferably at least 20 or even 30) of which have distinct core peptides. If the multi-ligand binding receptor is an MHC class I or an MHC class II receptor, and the ligands have been extracted from a single allotype, at least 40 of the ligands in the profile will preferably have distinct core peptides, and more preferably at least 50 (e.g., at least 70 or at least 100). Even more preferably, the method of the invention provides for the generation of a profile comprising at least 200 ligands having distinct core peptides, and most preferably at least 500. If the profile includes at least 70 ligands having distinct core peptides, such ligands may be extracted from one or more different multi-ligand binding receptors. In many cases, the profile will represent ligands extracted from two or more different multi-ligand binding receptors.

In preferred embodiments, the profiles represent a total of at least 50, preferably 500, more preferably 1000, and most preferably 5,000 through 10,000 ligands. These numbers include peptide or protein members with overlapping amino acid sequence, i.e., which do not necessarily have distinct core peptides. In preferred embodiments, the ligands represent at least 10% of the proteins expressed in the cell of interest; in more preferred embodiments, the ligands represent at least 20%, for example at least 30%, at least 50%, or even at least 80% of the proteins expressed in the cell.

In preferred embodiments, the multi-ligand binding receptor is an MHC class I or an MHC class II receptor, or a multi-ligand binding domain thereof. In alternative embodiments, the multi-ligand binding receptor is a chaperone, e.g., calnexin, calreticulin, BIP, grp96, and/or grp94, or a multi-ligand binding domain thereof. In alternative embodiments, the multi-ligand binding receptor is a chaperonin, or an hsp, *e.g*., hsp60, hsp65, hsp70, hsp90, and hsp25, or a multi-ligand binding domain thereof. In an again alternative embodiment, the multi-ligand binding receptor is a proteasome complex or a multi-ligand binding component or domain thereof. In an again alternative embodiment, the multi-ligand binding receptor is another component of the ubiquitin pathway, e.g., an E2 ubiquitin carrier protein (e.g., CDC34), an E3 ubiquitin ligase (e.g., cyclosome or components thereof, G1/SKP1/Cullin/F-box complex, E3α, hectdomain protein), an unfoldase, an hsp100, or a multi-ligand binding component or domain of any of the above. In an again alternative embodiment, the multi-ligand binding receptor is a mannosidase or a N-glycanase, or a multi-ligand binding domain thereof. In an again alternative embodiment, the multi-ligand binding receptor is a trafficking or retention protein, e.g., the KDEL receptor, the mannose receptor, or a multi-ligand binding domain thereof. In again alternative embodiments, the multi-ligand binding receptor is not an MHC class I or MHC class II receptor. In most preferred embodiments, the multi-ligand binding receptor is an allelic variant of an H-2 receptor, or an HLA receptor, such as HLA class II, *e.g.,* HLA-DR, HLA-DQ, or HLA-DP, or HLA class I, *e.g.,* HLA-A, HLA-B, HLA-C, HLA-C, HLA-E, HLA-F, or HLA-G receptor, or a multi-ligand binding domain thereof, or a combination of two or more of them.

The multi-ligand binding receptors are isolated using techniques generally known in the art. An important aspect for the choice of the procedure employed for the isolation and purification of the multi-ligand binding receptor(s) is that this step is performed under such conditions and in such manner that the bound repertoire of peptides remains associated with the receptor during the process.

In one embodiment of the invention, the multi-ligand binding receptors are isolated by immuno-affinity purification. Depending on the multi-ligand binding receptor to be isolated, monoclonal or polyclonal antibodies directed to suitable domains of the multi-ligand binding receptor are employed. Typically, the antibody is a monoclonal antibody. Further, the antibody has an affinity and specificity for the respective multi-ligand binding receptor that allows purification of the multi-ligand binding receptors under operational conditions (Smith *et al.,* 1989, *Proc. Natl. Acad. Sci. USA* 86:5557-5561; Gorga *et al.,* 1986, *J. Biol. Chem.* 262:16087-16094). Suitable antibodies include ones directed to an MHC class I receptor allotype, an MHC class II receptor allotype, a chaperonin, a calnexin, a calreticutin, a mannosidase, a N-glycanase, a BIP, a grp96, a grp94, hsp60, hsp65, hsp70, hsp90, or hsp25, an E2 ubiquitin carrier protein, CDC34, an E3 ubiquitin ligase, a cyclosome, a G1/SKP1/Cullin/F-box complex or individual components of such, an E3α, a hectdomain protein, an unfoldase, hsp100, a 26S proteasome complex, a 20S proteasome complex, or a trafficking or retention protein.

Alternatively, the multi-ligand binding receptor(s) are purified using ConA Sepharose or N-ion exchange chromatography. Such a purification method was successfully used by Blachere *et al.* to purify heat shock protein-peptide complexes. Blachere *et al*., 1997, *J*. *Exp. Med.* 186:1315-1322. In again another alternative embodiment, the multi-ligand binding receptor(s) are isolated using a series of different purification steps, for example an immunoaffinity purification step followed or preceded by one or several conventional purification steps. The skilled artisan will know what series of steps to apply to isolate the multi-ligand binding receptors at a sufficiently high level of purity. Generally, the multi-ligand binding receptor(s) are isolated and purified to a level of purity that is sufficient to achieve reproducible results. The skilled artisan will appreciate what conditions and techniques will permit the bound repertoire of ligands to remain associated with the receptor during the process.

After the multi-ligand binding receptor is purified, the bound repertoire of ligands is released from the receptor and separated using techniques generally known in the art. In one embodiment of the invention, the repertoire of ligands is isolated and separated using HPLC, for example, anion-exchange chromatography, cation-exchange chromatography, reversed-phase chromatography, normal phase chromatography, or hydrophobic-interaction chromatography. Alternatively, the repertoire of ligands may be isolated and separated using capillary electrophoresis peptide separation, for example, CE, AEC-CE, CZE, or CEC-CE.

The isolated ligands represented in the profile may be characterized according to a number of different physical and chemical parameters, including time of elution, actual mass, relative ionization or chemical structure or sequence. The parameters may differ with respect to the ligand separation technique applied. *See*, *supra.* In brief, depending on the separation technique applied, the physical separation profile may be according to the ligands' relative charge, hydrophobicity, hydrophilicity, mass, or hydration.

Generally, the profiles of the invention may be generated from any cell type of interest that expresses a multi-ligand binding receptor. Cells suitable for the generation of the profiles of the invention include, but are not limited to, cells derived from organ systems of interest, including heart, kidney, lung, spleen, brain, blood, skin, liver, thymus, intestine, or colon. The cells may be derived from various tissue types of interest, including muscle tissue, neuronal tissue, epithelium, endothelium, fat tissue, ovarian tissue, testicular tissue, skeletal tissue, bone marrow tissue, cardiac tissue, or mammary tissue. Cells suitable for the generation of the profiles may be derived from the hematopoietic system, such as pluripotent stem cells, T-cells, B-cells, macrophages, dendritic cells, P_{MNS}, mast cells, eosinophils, megakaryoctes; or any other primary cells (e.g., epithelial or endothelial cells) derived from a subject, e.g., a diseased or healthy human or animal or other organism; or any cell line of interest.

Typically, the profile is generated from a sample of isotypic cells, i.e., cells of identical origin and/or treatment. Most ideally, the cells are separated to substantial purity, i.e., essentially free of any other "contaminating" cell types prior to the generation of the profile. The cells of interest may be separated from any contaminating cell types using methods generally known in the art, including immunopurification using antibodies against cell surface proteins specific for the particular cell type of interest, magnetic beads, complement lysis, adherence to certain materials such glass or plastic, discrimination by size, cell density, FACS sorting, or cloning. In preferred embodiments, the sample contains cells of interest at a purity of at least 95%, more preferably at least 98%, even more preferably at least 99%, and most preferably at least 99.9% free of other types of cells. In cases where it is impractical to isolate the cells of interest with substantial purity, or where preferred for other reasons, the profile, of course, may be generated from a defined collection of cells, including the cells, tissue or organ of particular interest.

The choice of multi-ligand binding receptors used for the isolation of ligands largely depends on the particular cell of interest from which the profile is to be generated, and the experimental question. For example, for the generation of a profile representing ligands reflecting a substantial portion of all proteins expressed in a B-cell or a macrophage (e.g., all or as close to "all" proteins expressed in the cells as possible), suitable multi-ligand binding receptors include allotypes of MHC class I and MHC class II receptors, or a combination thereof. For the generation of such complex profiles for a non-professional antigen-presenting cell, MHC class I receptors will generally be a good choice, as most nucleated cells express MHC class I receptors. Expression of MHC class II receptors can be induced in many cells which do not normally express them, by treating the cells with γ-interferon or other agents known to those in the field of immunology. In cases where the experimental goal is to generate a profile that corresponds to a more specific set of ligands, other types of multi-ligand binding receptors may be preferred. For example, where the goal is to generate a profile reflecting cell cycle components present in a cell or tissue type of interest, a multi-ligand binding receptor specifically binding to cell cycle components may be the choice. The skilled artisan will know how to determine what the suitable multi-ligand binding receptor(s) for the isolation of predetermined ligands, i.e., ligands selected according to a specific set of parameters, of a particular cell type of interest would be.

Expression and/or presence of the different multi-ligand binding receptors in a cell type may be determined using methods generally known in the art, including but not limited to mRNA hybridization techniques using nucleic acid probes specific for various multi-ligand binding receptors, such as Northern blots, *in situ* hybridization, dot blots, RNase mapping, S1 nuclease mapping, or RT-PCR, or immunohistological techniques using antibodies specific for the multi-ligand receptor binding protein, such as Western blots, FACS analysis, immunoprecipitation, ELISA, or *in situ* immunostaining. *See, e.g*., Sambrook *et al*., 1989 Molecular Cloning: A Laboratory Manual 2nd ed. Cold Spring Harbor Laboratory Press; Ausubel *et al.,* Current Protocols in Molecular Biology, Greene Publishing Associates and Wiley Interscience, N.Y. (current edition); Harlow and Lane (Harlow, E. and Lane, D., 1988, "Antibodies: A Laboratory Manual", Cold Spring Harbor Laboratory Press, Cold Spring Harbor, New York).

### Generation of Profiles of Ligands Differentially Present in Two or More Different Cells of Interest

In one embodiment, the invention relates to a method of generating a differential or "subtraction" profile of ligands which are differentially present in two or more different cells of interest. Generally, this method involves generation of a first pool of ligands extracted from a first sample, and a second pool of ligands extracted from a second sample, and the identification of ligands that are present in said first pool of ligands and absent in said second pool of ligands, or vice versa, to form a differential profile of ligands. The first pool of ligands and a second pool of ligands are generated by essentially the same procedures as described above. *See, supra.* In brief, a first and a second pool of ligands are generated by isolating one or multiple types of multi-ligand binding receptors from a first cell of interest and a second cell of interest, respectively, under conditions that preserve association of the bound ligands; extracting the ligands bound to the receptor(s); and characterizing the ligands according to selected parameters, such as amino acid sequence, HPLC profiles (anion-exchange, cation-exchange, reversed-phase, normal phase, hydrophobic-interaction chromatography), capillary electrophoresis profiles (CE, AEC-CE, CZE, or CEC-CE), and mass spectrometry profiles (MALDI-TOF/MS, FTMS, ESI-TOF, MALDI-ITMS, ESI-Quadropole MS, ESI-Quadropole/TOF-MS, ESI-Sector MS, FAB-MS, or ESI-ITMS), and resulting properties. Subsequently, those ligands are identified and/or isolated that are present in the first pool of ligands and absent in the second pool of ligands, or vice versa, according to any of the parameters employed for the characterization of the ligands of the first and the second pool.

Generally, the first and the second samples may comprise any cell, tissue, or organ type of interest. In one embodiment, the sample comprises cells that are not professional antigen presenting cells. In a specific embodiment, the cells are not B-cells. In another specific embodiment, the cells are not macrophages. In an alternative embodiment, the cells are professional antigen presenting cells.

In preferred embodiments, the ligands represented in the differential profile are present in the first pool of ligands, but absent in the second pool of ligands, or vice versa. In other embodiments, the ligands represented in the differential profile are more abundant at detectable levels in the first pool of ligands than in the second pool of ligands, or vice versa.

In accordance with the above outlined methods and procedures, a differential profile of the invention consists of a subset of ligands that is differentially present in two (or more) distinct cell types, disease stages, developmental stages, metabolic stages, cell cycle stages, treatment regimens, etc., of interest. As such, the differential profiles represent a repertoire of ligands that may directly or indirectly be involved in the different cellular phenotypes or behavior. Consequently, the differential profiles provide a valuable tool for the characterization of cell-type and/or phenotype-specific protein expression, and for the identification and/or the isolation of known or novel gene products and their respective coding sequences that are potentially involved in biological processes, such as developmental processes, establishment and progression of disease, predisposition to disease, organ development, signal transduction, differentiation, neurogenesis, etc., or in response to environmental factors or treatments.

### Characterization of Cell-Specific Protein Expression

In one embodiment of the invention, ligands, in particular peptide or protein ligands, expressed differentially in two or more different cell sources are identified and isolated. The polypeptide ligands identified as differentially expressed may be further characterized by determination of their chemical structure: i.e., sequence. Thus, the present technique provides for the characterization of differential expression, e.g., the presence or absence, of gene products encoded by known genes and/or ESTs with unknown function. The methods and tools of the present invention thus provide an easy and efficient way to assign to previously identified genes or gene products a putative function and/or involvement or association with a particular developmental pathway, metabolic pathway, or disease stage. With this information, new targets for the development of gene therapy approaches and drug development may rapidly be identified.

If the nucleic acid sequence or a fragment thereof, e.g., in the form of an EST, cannot be found in any of the available databases, the sequence of the gene encoding the protein of interest may be identified using standard techniques.

### Identification of New Genes

In one embodiment, the methods and tools of the present invention are used for the identification of novel proteins and the genes which encode them. Specifically, if the nucleic acid sequence encoding a particular protein or peptide of interest (or the peptide sequence itself) does not match any known sequence in existing databases, the corresponding gene may be cloned using degenerate primers derived from the EPT sequence.

The skilled artisan will appreciate that a number of methods are known in the art to identify and isolate genes or cDNAs using amino acid information, and will know how to identify and practice such methods. See, for example, Sambrook *et al*., 1989 Molecular Cloning: A Laboratory Manual 2nd ed. Cold Spring Harbor Laboratory Press; Ausubel *et al*., Current Protocols in Molecular Biology, Greene Publishing Associates and Wiley Interscience, N.Y. (current edition).

### Generation of Databases of EPT Profiles

The generation of profiles as described above allows for the creation of a highly specific "fingerprint" of EPTs in a given cell of interest. As discussed *supra*, the peptide profiles may be displayed, dependent on the number of parameters chosen, in multi-dimensional coordinates in multi-dimensional space. An important aspect of the invention is to provide databases to manifest, store, and display the multi-dimensional information regarding the mass/charge, hydrophobicity, hydrophilicity, relative intensity, relative ionization, structure, sequence, function, cellular compartment location etc. See, for example, Fig. 6.

The databases of the invention are used for a number of applications. First, they are used as a reference point for a human patient's or animal's sample for the diagnosis of disease, progression of disease, and predisposition for disease. For example, if a disease is associated with changes in protein composition in certain cells, organ systems, cell sources, or tissue types, a suitable patient sample may be used to generate a protein profile according to the methods of the invention, and compared with profiles of corresponding samples of normal (non-diseased) and/or diseased origin to assess presence or absence of, progression of, and/or predisposition to the particular disease in question. A large number of diseases may be diagnosed this way, including diseases for which particular aberrations in protein expression are known, including, but not limited to metabolic diseases that are associated with lack of certain enzymes, proliferative diseases that are associated with aberrant expression of, e.g., oncogenes or tumor suppressors, developmental diseases that are associated with aberrant gene expression, etc. Furthermore, the methods and tools of the invention allow for the diagnosis of diseases or other aberrations simply based on pre-determined differences in EPT profiles. Thus, if it is pre-determined that a given disease of interest is associated with certain changes of the EPT profile of a particular type of cell, tissue, cell source, or organ system, a human patient or animal may be diagnosed simply based on its individual profile when compared to the profiles provided by the databases in accordance with the invention.

Second, the information stored in the databases of the invention may be used to identify novel or known genes and their products that are involved in the manifestation of, progression of, or predisposition to any disease of interest, and with the development of symptoms of a particular disease. For example, EPT profiles of a diseased organ, tissue or cell type may be generated and compared with the corresponding profile counterpart obtained from a non-diseased sample. Differences in the profile may be identified, and individual EPTs that are differentially present in the diseased vs. the non-diseased sample may be identified and isolated for further analysis. *See, supra.* The identified differences in the EPT profiles are useful for future diagnosis of the disease or aberration. The obtained information may further be used to identify and isolate the differentially expressed gene(s), which in turn may be useful for the development of targeted treatment of the disease.

The database could store three categories of data respectively representing (a) ligand profiles, (b) cell sources, and (c) receptor types. The ligand profile information could contain a variety "multidimensional" data including the kinds of information discussed earlier. The ligand profiles would typically include information that uniquely identifies protein fragments, e.g., mass spectral data or protein sequences. The information about receptor types could likewise be in a variety of forms, e.g., name, sequence, or biochemical characteristics. Characteristics of different cell sources that could be stored in the database are indicated in the definition of cell sources above.

Instances (e.g., values) of each of the categories of information would be used for storing records in the database. An instance could be, for example, a particular ligand profile, or a particular cell source, or a particular receptor type.

Each of the categories of information could be broken into subcategories. A cell source could be broken into cell sub-sources. For example, a cell source for diseased cells could include sub-sources for cancerous and diseased but non-cancerous cells, or for different stages of cancer development, and so on.

In some kinds of databases, the categories could be implemented as fields within tables and instances could be values in records belonging to the tables.

In any event, the database would define associations among instances of the three categories of data. For example, the database could associate a specific instance of a ligand profile with an instance of a receptor type and with an instance of a particular cell source.

The associations enable finding instances of data of any one of more of the categories based on their associations with instances of data of another one or more of the categories. For example, a known receptor type could be used to find one or more ligand profiles or cell sources. A wide variety of query strategies would be made possible by the stored information.

The cell sources can be types of cells, cell conditions, genetic background, identities of individuals from which the cells were derived, states of perturbation, or developmental states. By "condition", we mean such variables as culture conditions, general health or age of the animal from which the cells were derived, transgenic vs. nontransgenic, transfected vs. nontransfected, virus- or prion-infected vs. noninfected, etc. By "perturbation", we mean experimental manipulation of the cells, such as treatment with a particular compound vs. nontreatment or treatment with a different dosage. The stored information about ligand profiles could include mass spectral data.

One use of the database would be to find ligand profiles associated with selected cell sources and receptor types. Another use would be to find two ligand profiles and determine a difference between them.

More generally, the database could be used to support a wide variety of experiments in which a ligand profile associated with cells is identified. Based on the ligand profile, a query is directed to the database to derive a cell source, or a ligand profile and an associated cell source. Several examples of such experiments follow.

Cells may be treated using a candidate drug regimen and the database may be queried for a cell source representing a different treatment of similar cells (e.g., a different drug or no drug, or the candidate drug used in a different way). The candidate drug may bind specifically to a particular protein, permitting isolation of cells which express that protein; the query may derive information about cell sources that express the particular protein.

An animal may be treated using a test compound regimen and a ligand profile may be determined. The database is then queried for a cell source that represents cells of the same animal, but prior to treatment with the test compound, or for a cell source that represents cells from another animal, before or after treatment with the same or a different test compound.

Cell development may be controlled and the determined ligand profile may be associated with the development of the cell. The database may be queried for a cell source that represents a stage in development different from that of the cell source of the cells of the experiment.

An expression vector may be introduced into cells of a cell source and the determined ligand profile may be associated with the effects of the expression vector. The database may be queried for a cell source which lacks the expression vector used in the experiment.

The response of cells to pharmacological compounds may be observed and the determined ligand profile may be associated with responsiveness or non-responsiveness to the compound. The database is queried for a cell source that is phenotypically different from the cell source of the cells of the experiment (e.g., the same cells but not treated with the pharmacological compound).

For use in these and other kinds of experiments, the database could be distributed on a medium such as a CD-ROM, or could be queried by an online connection from a searcher to the location where the database is stored and maintained. The database could be made available on the World Wide Web to permit online searching using web browsers. Information generated by querying of the database could form the basis of services to be provided by an owner or user of the database to third parties.

For example, in one kind of service a cell source, a receptor type, or a ligand profile of interest would be identified. Based on the identified cell source, receptor type, or ligand profile, the database would be queried to derive information about cell sources, receptor types, or ligand profiles that relate to the cell source, receptor type, or ligand profile of interest.

In another service approach, a vendor would receive cells of a cell source from a customer. The vendor would generate a ligand profile from the cells. Based on the ligand profile and the cell source, the vendor would query a database to derive information about cell sources, receptor types, or ligand profiles that relate to the received cell source and the generated ligand profile. The vendor could provide the service from a database controlled by the vendor who could use a database available from a third party.

### Applications of EPT Profiles

### Generating EPT Profiles for Different Developmental, Metabolic or Disease Stages of a Given Type of Cell

Ligand profiles for cells of different developmental, metabolic or disease stages are generated and compared to identify differences in protein or gene expression.

In one specific embodiment, ligand profiles of diseased vs. normal cell types are generated. For example, the profiles of a cancer cell and non-cancerous cell derived from the same genetically matched tissue may be generated and compared. Proteins differentially expressed in diseased and non-diseased cells can conveniently be identified, and their involvement in disease development and progression analyzed by methods well known in the art. In this way, new targets for the treatment of the disease are efficiently identified.

Alternatively, ligand profiles of cells of different developmental stages are generated and compared. For example, profiles of embryonic cells and adult cells derived from genetically matched tissue may be generated and compared to identify genes and their products that play a role in developmental processes, and that may be useful for the development of, e.g., novel gene therapy or other therapeutic approaches for the treatment of developmental disorders.

In another specific embodiment of the invention, EPT profiles of (a) cells infected with a selected pathogen, e.g., microorganism, virus, retrovirus, or prion, and (b) corresponding non-infected cells are generated and compared to identify genes and gene products that are turned on or off in response to the infection. Alternatively, instead of being infected, the first cell can be made to take up a foreign protein or immunogenic substance, etc. This approach allows one, e.g., to identify factors produced by the cells in response to infection or introduction of the foreign substance that could be useful for therapeutic purposes.

In another example, ligand profiles from cells derived from individuals having a selected genetic disorder and individuals that do not have such disorder are generated and compared. Preferably, samples from affected and non-affected family members are used for the generation of the profiles. Depending on the particular genetic disorder chosen, cell or tissue types that are known to be affected by the particular genetic disorder are studied. In many cases, profiles of various cell and/or tissue types will be generated and compared. This embodiment of the invention allows one to identify genes and proteins associated with a genetic disorder. The information obtained may be useful for the development of gene therapy and other therapeutic approaches and for the development of targeted drugs that interfere with the expression of genes or activity or stability of gene products that are involved in the symptoms of the genetic disease. Furthermore, this embodiment of the invention allows selection of diagnostic targets for the identification of individuals predisposed for certain types of disease or disease symptoms.

### Generation of EPT Profiles Correlated to Response of a Given Cell Type to External Factors

In one embodiment of the invention, an EPT profile of a given cell type treated with an external factor is generated and compared to a profile of cells of the same type which have not been so treated, to identify differences in protein expression. The cells can be recombinant or native, a cell line or non-transformed cells, or isolated directly from an animal before and after treatment of the animal with the compound.

In one embodiment of the invention, EPT profiles of cells of a selected origin or nature that have been contacted with a growth factor, cytokine or hormone, and cells that have not been contacted with the substance, but otherwise treated the same way, are generated and compared. This allows identification of genes and gene products that are turned on or turned off in response to the growth factor, cytokine or hormone, which will give, e.g., valuable insight in cellular signal transduction pathways and regulation of protein expression.

Similarly, ligand profiles of cells that have been treated with or exposed to a polypeptide, small molecule, chemokine, or nucleic acid drug or drug candidate, and cells that have not been treated with or exposed to the substance, but have otherwise been treated the same way, are generated and compared. This allows one to identify the effects of the selected substance on protein expression in the cell, and is, for example, an excellent tool for the validation of particular drugs or the identification of drugs associated with expression of a selected gene or gene product.

In another example, ligand profiles of cells that have been exposed to a selected type of compound, e.g., a selected carbohydrate or group of carbohydrates, lipid or group of lipids, amino acid or group of amino acids, nucleotide or nucleoside or group of either, or vitamin or group of vitamins, and cells that have not been treated with the compound, but have otherwise been treated the same way, are generated and compared. This allows one to identify the effects of the selected compound on the gene and protein expression of the cell, and will give valuable insight into metabolic processes.

In another embodiment of the invention, ligand profiles of cells that have been treated with a selected nucleic acid, e.g., a selected antisense oligonucleotide, a ribozyme, an expression vector, a plasmid, an RNA, or a DNA, and cells that have not been treated with the nucleic acid, but have otherwise been treated the same way, are generated and compared. This allows one to identify the effects of the antisense oligonucleotide or other nucleic acid on the protein expression in the cell, and as such allows one to evaluate the efficacy or effect of the antisense oligonucleotide or nucleic acid.

Finally, ligand profiles of cells that have been subject to a selected stress condition, such as low or high temperature, hypoxia, deprivation of nutrients, such as glucose, amino acids, or other essential factors, or presence of a toxin, are generated and compared to an EPT profile generated in untreated controls. Differentially expressed gene products are identified in order to give valuable insight into factors involved in cellular stress responses. This aspect of the invention provides an extremely valuable and efficient way to determine and/or evaluate the effect of a selected compound on protein expression in the cell. The technique may furthermore be useful to verify a desired shut-down of certain enzymatic activities, *e.g.,* by distinguishing between phosphorylated and non-phosphorylated, or glycosylated and non-glycosylated, peptides and/or proteins. It can also be used to aid in pharmacological and/or toxicological assessment of potential new drugs, and in screening for such drugs.

### Generating EPT Profiles for Different Organ Systems

Ligand profiles of cells derived from different organs or organ systems may be generated and compared to identify differences in protein or gene expression. For example, ligand profiles of cells derived from lung, liver, heart, spleen, skin, brain, kidney, thymus, intestine, and/or colon can be generated and compared. Differentially expressed genes and proteins are thus identified. This aspect of the invention is useful to identify proteins that are involved in an organ's particular physiological function.

In another embodiment of the invention, EPT profiles of selected tissue or cell types, e.g., muscle, endothelium, epithelium, neuronal, fat, ovarian, testicular, blood, bone marrow, and/or mammary tissue, etc., are generated, compared, and differentially expressed proteins identified. This will give valuable insight into a protein's involvement in a tissue or cell type's physiological function.

### Generating EPT Profiles for Expression Studies in Standard Cell Lines

Ligand profiles of cells derived from differentially engineered standard cell lines can be generated and compared to identify differences in protein expression.

For example, EPT profiles of standard cell lines that have been engineered to express/overexpress one or several selected recombinant genes, *e.g.,* genes encoding a selected growth factor receptor or other signal transduction component, transcription factor, oncogene, apoptosis-inducing gene, etc., are generated and compared to EPT profiles prepared from a reference cell line of the same origin, but which does not carry and express the selected recombinant gene. Differentially expressed genes and gene products are identified. This will allow one to identify the impact of the overexpressed gene on the expression of other polypeptides in the cell.

### The Use of Ligand Profiles to Characterize Gene Expression Patterns in Transgenic and Knockout Animals

A ligand profile of a selected cell or tissue type derived from a transgenic or knockout animal is generated and compared with a profile of the same cell or tissue type of an isogenic but non-transgenic animal, to identify differences in protein or gene expression. This aspect of the invention is a valuable tool for the testing and verification of actual gene knock-outs and the testing of gain and loss of protein expression in transgenics. This aspect further allows one to characterize the effect of a gene's loss or gain of function on expression patterns in general.

### The Use of EPTs to Assist in Positional Cloning Efforts

EPT profiles can also be used to assist in positional cloning efforts. For example, EPT profiles of YACs, PACs, minichromosomes or cosmids or other vehicles comprising large pieces of unknown nucleic acids may be generated in order to identify clones that encode a protein of interest.

In one aspect, a nucleic acid encoding one or several selected multi-ligand binding receptor(s), or a soluble form of the receptor, operatively linked to nucleic acid elements driving transcription and translation, is cloned into a minichromosome, YAC, PAC, cosmid or other vehicle that contains a portion of the genome of a species of animal or other organism of interest. The YAC, PAC, minichromosome, cosmid or other vehicle is then introduced into and expressed in suitable cells. The selected multi-ligand binding receptors of the cells are purified, and the peptide or protein ligands are extracted, separated and characterized as described above. Gene products of interest that are encoded by the nucleic acid are identified. General protocols for the formation of YACs, minichromosomes, and cosmids, and for generation of cells expressing the same, etc., can be found in Ausubel *et al., supra.* Additional information on YACs can be found in Montanaro *et al*., 1991, *Am. J. Hum. Genet.* 48:183-194; Somerville, 1991, *Mol. Gen Genet*. 226:484-490; Coulson *et al*., 1988, *Nature* 335:184-186; Green and Olson, 1990, *Science* 250:94-98; Kai *et al.,* 1990, *FEBS Letters* 275:77-82: Imai and Olson, 1990, *Genomics* 8:297-303; Okazaki and Hayashizaki, 1997, *Methods* 13:359-377; Parimoo, 1997, *Mol. Biotechnol.* 8:255-268; Forster and Rabbitts, 1993, *Oncogene* 8:3157-3160; Feingold et al., 1990, *Proc. Natl.* Acad. Sci. *USA* 87:8637-8641.

In an alternative aspect, large pieces of uncharacterized DNA (mini-chromosomes, cosmids, PCAs, YACs, etc.) are introduced into cells expressing one or several selected multi-ligand binding receptor(s), to generate EPT profiles of the gene products expressed by the uncharacterized piece of DNA. Comparison of the ligand profile from a given multi-ligand receptor with the corresponding profile from a cell not expressing the large piece of uncharacterized DNA yields information about what is expressed on the transfected segment of DNA. To the extent that expression of any particular gene on the uncharacterized DNA is cell-specific, carrying out this method using a variety of cell types may yield additional information about the identity of the genes on the uncharacterized DNA. For general protocols and references, *see, supra.*

### The Use of the Multi-Ligand Binding Receptor System to Sort Exogenous Proteins

The multi-ligand binding receptor systems may also be used to sort and isolate exogenous proteins or peptides *in vitro* and/or to determine the multi-ligand binding receptor's EPT binding properties.

For example, recombinant or purified multi-ligand binding receptors are employed to determine the EPT profile of a specific cell, tissue or organ type of interest. For example, recombinant and/or purified multi-ligand binding receptors of a selected type or combination of types are exposed to proteins or peptides (as random or predetermined degradation products of such proteins) derived from, *e.g.,* an expression library of a source of interest. For example, mRNA derived from a cell, tissue or organ type of interest may be isolated and reverse transcribed into cDNA. The cDNA, representing the repertoire of nucleic acids that could be expressed as proteins in that particular cell, tissue, or organ type of interest, is then, either through generation of an expression library (Sambrook *et al*., 1989, *supra;* Ausubel *et al*., *supra)* or through direct *in vitro* transcription and translation (Sambrook *et al*., 1989, *supra*; Ausubel *et al*., *supra),* expressed as a corresponding repertoire of proteins. Depending on the multi-ligand binding receptor system used, the proteins may be incubated with the multi-ligand binding receptor directly, or may be fragmented into peptides, e.g., by proteolytic digestion, of a size that is known to be the preferred binding partner of the multi-ligand binding receptor, and then incubated with same under suitable conditions known to an artisan skilled in the art. The receptor/ligand complexes are then isolated, and the ligands extracted, separated, and characterized as described above. This approach may be particularly preferred in cases where the cell, tissue or organ of interest does not express the selected multi-ligand binding receptor(s) in sufficient amount. For example, brain tissue appears to express only small amounts of MHC class I and II receptor molecules; with this <i>in vitro </i> approach these receptors may still be employed to generate complex EPT profiles of brain tissue or brain cells.

In another specific embodiment, this <i>in vitro </i> approach is used to determine the binding specificity of a selected multi-ligand binding receptor of interest. For example, recombinant or purified multi-ligand binding receptors of interest, are exposed to peptide libraries under conditions appropriate to facilitate binding of the ligands. The receptors are isolated and purified, and the associated repertoire of peptides is extracted and characterized. This allows one to identify, isolate and characterize the repertoire of ligands binding to a multi-ligand binding receptor of interest, to obtain an artificial "fingerprint" of the particular multi-ligand binding receptor. Identifying the sequence of each member of the artificial fingerprint allows one to map the potential pool of ligands binding to a multi-ligand binding receptor of interest. Any sort of peptide or protein library may be used for the practice of this embodiment of the invention; however, very complex synthetic peptide libraries are preferred.

The examples below explain the invention in more detail. The following preparations and examples are given to enable those skilled in the art to more clearly understand and to practice the present invention.

### EXAMPLES

### Example 1: Purification of Multi-Ligand Binding Receptor/ Ligand Complexes in a Rapid and Reproducible Manner

The following experiment shows an example of a rapid and reproducible purification of multi-ligand binding receptor/ligand complexes according to the invention. More specifically, EPT complexes of HLA-A*0201 and HLA-DR*0401/1301 from 20 g (Fig. 1A) and 22 g (Fig. 1B) of the human lymphoblastoid B cell line, JY, have been purified using an automated, in-line, immunoaffinity chromatography purification strategy. The chromatograms represent the protein content as detected by UV absorbance at 280 nm on the y-axis and the time in minutes on the x-axis.

***METHODS*.** The human cell line JY was grown to a final cellular density of ~10⁶/ml. Cells were harvested by sedimentation and the decanted pellets were weighed to determine the cellular mass present, then frozen at -80°C until just prior to lysis. The cell pellet was resuspended in 10 mM Tris-HCl, 1 mM dithiothreitol (DTT), 0.1 mM phenylmethylsulfonylflouride (PMSF), pH 8.0 at 4°C, and lysed in a homogenizer. The nuclei were removed by sedimentation at 4,000x g for 5 minutes and the pellets washed and repelleted until the supernatants were clear. All the supernatants were pooled and the membrane fraction harvested by sedimentation at 175,000x g for 40 minutes. The pellets were then resuspended in 10 mM Tris-HCl, 1 mM DTT, 1 mM PMSF, 4% Nonidet P-40 (NP-40). The unsolublized membrane material was removed by sedimentation at 175,000x g for 2 hours, and the NP-40 soluble supernatant fraction used for subsequent receptor:EPT purification. Multi-modal protein purification using HPLC columns was achieved by coupling the chromatographic sorbents in series with automated switching valves, which direct the protein:EPT complex containing effluent to subsequent columns in the sequences. The first three coupled columns were connected directly in series and acted together as a single pre-clearing column using high strength large throughpore perfusion sorbents (6000-8000 Å throughpores and 500-1000 Å diffusive pores, 50 *µ*m) coated and crosslinked with a hydrophilic stationary phase covalently attached to Protein A (POROS A^{™} sorbent). These columns were designed to remove any proteins which adsorb non-specifically to the base sorbent or to the constant domain of murine monoclonal antibodies. Column 1 was an unmodified Protein A sorbent, column 2 was Protein A conjugated with normal mouse serum, and column 3 was Protein A conjugated with bovine serum. The pre-clearing columns were followed in series by three independent immunoaffinity columns of Protein A coupled with specific monoclonal antibodies: anti-HLA-A2 (mAb BB7.2: Parham and Brodsky, Hum. Immunol. 3:277-299, 1981); anti-HLA-A/-B/-C (mAb W6/32: available from the American Type Culture collection (ATCC)); and anti-HLA-DR (mAb LB3.1: Knudson and Strominger, Hum. Immunol. 15:150-163, 1986). The immunoaffinity columns were then extensively washed using 50 column volumes of 20 mM MOPS/140 mM NaCl/0.1% DOC/0.05% NaN₃ at pH 8.0 followed by 100 column volumes of 10 mM Tris/0.1% DOC/0.05% NaN3 at pH 8.0. The receptor:EPT complexes were eluted independently from each immunoaffinity support using 3.5 column volumes of 50 mM carbonate/0.1% DOC/0-05% NaN₃ at pH 11.5. The peak labeled 1 in each of Figs. 1A and 1B represents the HLA-A*0201:EPT complex elution profile, while the peak labeled 2 represents the HLA-DR*0401/1301:EPT complex elution profile.

### Example 2: Purity Analysis of Multi-Ligand Binding Receptor/Ligand Complexes

The following example is an SDS-polyacrylamide gel electrophoresis purity analysis of the receptor/EPT complexes purified from the human B lymphoblastoid cell lines LG-2 and JY using techniques as described in *Example 1.*

***METHODS.*** Aliquots of vacuum-dialyzed receptor:EPT complex material isolated as described in *Example 1* and corresponding to between 2 and 5 *µ*g of protein were boiled for 5 minutes, separated on a 12% polyacrylamide gel, and stained using Coomassie Blue. Samples run in lanes 2-4 were purified from the human cell line LG-2 whereas lanes 5-7 were purified from the human cell line JY. The results are depicted in Fig. 2, in which the samples are labeled as follows: Lane 1: Molecular weight markers; Lane 2: HLA-A*0201; Lane 3: HLA-B*2701 and HLA-Cwl; Lane 4: HLA-DR*0101; Lane 5: HLA-A*0201; Lane 6: HLA-B*0702 and HLA-C*0701;
Lane 7: HLA-DR*0401 and HLA-DR*1301.

### Example 3: Reversed-Phase Separation Profiles of Two Independent HLA-A*0201:EPT Preparations

The following example illustrates generation of reversed-phase separation profiles of two independent HLA-A*0201:EPT preparations, obtained as described in *Example 1.* The two overlaid chromatograms shown in Fig. 3 represent the EPT repertoire as detected by UV absorbance at 210 nm. They are overlaid to demonstrate the reproducibility of the separation necessary for EPT profile comparisons.

***METHODS.*** Purified HLA-A*0201:EPT complexes (310 *µ*g and 340 *µ*g respectively) were acid extracted using 10% acetic acid and heated to 70°C for 5 minutes. The released EPT repertoires were separated from the denatured protein by ultrafiltration using a 10 kDa filtration device. The isolated EPT repertoires were fractioned based on relative hydrophobicity using a silica based C₁₈ support (300 Å, 5 *µ*m). The EPT repertoire was eluted using a non-linear buffer A/buffer B gradient protocol at a constant flow rate of 50 *µ*l/min: 0-63 minutes 5%-33% buffer B; 63-95 minutes 33%-60% buffer B; 95-105 minutes 60%-80% buffer B, where buffer A is 0.06% TFA/5% acetonitrile/H₂O and buffer B is 0.055% TFA/5% H₂O/acetonitrile. The chromatographic analysis was monitored by UV absorbance at multiple wavelengths (210 and 277 nm) to identify peptide bonds and EPTs containing conjugated delocalized π-electrons (aromatic amino acids). The more hydrophobic individual ligands elute later in the gradient with increased percentages of organic modifier. The results are depicted in Fig. 3.

The flow stream was interfaced with a 50:1 micro-fraction MALDI-TOF/MS sample plate collector, split to allow simultaneous sample collection and MALDI-TOF/MS sample preparation. In this manner, 2% of each fraction was immediately prepared for mass analysis while the remaining 98% of each fraction was collected and stored for future screening.

### Example 4: Mass Analysis of Single Isolated Fractions from Two Receptor : EPT Preparations

The following example describes mass analysis of single isolated fractions from two receptor:EPT preparations. Receptor:EPT isolation and EPT separation was accomplished for HLA-A*0201 and HLA-DR*0401 from the human cell lines JY and Priess, respectively, using methods as described in *Example 1* and *Example 3.* Representative mass analyses for selected RP-HPLC fractions are illustrated in Figs. 4A and 4B, respectively. Fig. 4A is the mass analysis spectrum for the complex mixture of individual EPTs found in RP-HPLC fraction 56, extracted from the HLA-A*0201 of cell line JY. Fig. 4B is the mass analysis spectrum for the EPTs found in RP-HPLC fraction 37, extracted from the HLA-DR*0401 of cell line Priess. The y-axis displays the relative ionization of each EPT, and the x-axis displays the mass-to-charge ratio (m/z) for the single charged species.

***METHODS**.* Samples isolated as described in *Example 3* were automatically collected onto MALDI-TOF/MS samples plates as described in *Example 3*. To each fraction, 0.5 *µ*l of UV absorbing matrix was added and allowed to crystallize under ambient room conditions. Samples were then analyzed on a research grade MALDI-TOF mass spectrometer in the reflectron mode of operation. Mass spectra were collected using a 20 kV accelerating voltage, 100 ns delay time (delayed extraction), and nitrogen laser at 337 nm, with optimal laser intensities, averaging the ion signals from 80 individual laser shots.

### Example 5: Determination of the Cellular Source Protein Represented by Individual EPTs

The following example illustrates the identification of the cellular source protein represented by individual EPTs. Specifically, the cellular source protein of each EPT can be determined by fragmentation of the EPT ion and subsequent sequence analysis followed by related EST sequence or other sequence database comparison. Fig. 5A depicts the post-source decay/collisional-induced dissociation spectrum of an individual EPT from the fractionation illustrated in Fig. 4B (m/z=1957.8). Fig. 5B shows a sequence analysis based on the parent ion mass, the daughter ion fragments, and the immonium ion composition. Fig. 5C depicts identification of related EST sequences. The amino acid sequence determined in Fig. 5B was used to perform a blastin search of the non-redundant GENBANK+EMBL+DDBJ EST divisions using the NCBI National Library of Medicine internet-based search engine. The resulting EST hits and translated reading frame matches and alignments are shown. This example demonstrates the ease with which EPT data can be cross referenced to EST data sets.

***METHODS**.* Composite post-source decay (PSD) and collision-induced dissociation (CID) MS/MS spectra were collected on a single stage reflector time-of-flight mass spectrometer (PerSeptive Biosystems Voyager Elite XL, Framingham, MA) utilizing timed ion selection (the timed ion gate was set for a m/z=1957.7) and a 20 kV accelerating voltage. The relevant focused fragment ions were acquired by sequentially reducing the parent ion's reflector mirror to source accelerating voltage ratio from 1.00 - 0.11. The composite spectrum was then analyzed, and the individual fragment ions combined with the parent ion mass were used to search the non-redundant Genpep database for possible peptide matches. As indicated in Fig. 5B, the cellular host protein from which the HLA-DR*0401:R4A3F37m1957 EPT is derived is HLA-A*0201.

### Example 6: Two-Dimensional Representation of a Human Lymphoblastoid B Cell EPT Fingerprint Extracted from the Human Receptor HLA-DR*1501

The following example describes a two-dimensional representation of a human lymphoblastoid B cell EPT fingerprint extracted from the human receptor HLA-DR*1501. The results are depicted in Fig. 6.

***METHODS**.* MALDI-TOF/MS analysis as described in *Example 4* was completed for the entire EPT repertoire isolated from the human lymphoblastoid B cell line, H0104. The precise EPT masses (m/z) from each spectrum were then recorded and plotted against the relative time of elution from the reversed-phase separation described in *Example 3*. The resulting "fingerprint" was then plotted as relative hydrophobicity (x-axis) versus m/z or size (y-axis) to result in the EPT profile of Fig. 6.

### Example 7: Generation of BiP-Specific Ligand Profile

The following describes how ligands would be isolated from BiP, a multi-ligand binding receptor that interacts with proteins in the ER.

There is evidence that BiP may interact with proteins to promote protein folding. Initial attempts at purifying BiP by gel filtration chromatography suggested that BiP interacts with several proteins in the ER. (Shin and Pastan, 1979, *Biochim. Biophys. Acta* 576:141.) Correct folding of many proteins translocated across the ER membrane requires disulfide bond formation. BiP is required for correct disulfide bond formation of the influenza hemagglutinin protein (Braakman *et al*., 1992, *Nature* 356:260-262), and interacts with disulfide bonded folding intermediates of prolactin (Kassenbrock *et al*., 1988, *Nature* 333:90-93). Furthermore, immunoprecipitation of T cell receptor proteins, immunoglobulin heavy chains and MHC class I heavy chains can precipitate BiP (Suzuki *et al.,* 1991, *J. Biol. Chem.* 114:189-204; Bole *et al.,* 1986, *J. Biol. Chem.* 102:1558. Thus, it is believed that BiP would be a useful multi-ligand binding receptor for the isolatation of ligands that are present in the ER.

ATP binding leads to the release of peptides or proteins by BiP (Munro and Pelham, 1986, *Cell* 46:291; Kassenbrock and Kelly, 1989, *EMBO J.* 8:1461). It was suggested that BiP interacts with incorrectly folded proteins and induces them to fold correctly by slow association and dissociation, driven by its weak ATPase activity. ATP hydrolysis may promote a conformational change in BiP that is translated to the substrate, resulting in substrate release, and over time, proper substrate folding. A role for ATP in the folding and unfolding of influenza HA within the ER was demonstrated by depleting cells of ATP (Braakman *et al*., 1992, *supra*). Thus, to isolate BIP in association with protein folding intermediates, or peptides, cells will be grown to the appropriate density and depleted of ATP by treatment with apyrase (Kassenbrock *et al*., 1988, *supra),* or incubation in conditioned media (Braakman *et al*., 1992, *supra).* The presence of Ca²⁺ has also been shown to increase substrate binding to BIP and enhance the ability to isolate BIP/substrate complexes (Kassenbrock and Kelly, 1989, *supra*; Suzuki et *al.,* 1991, *supra).*

Cells expressing BiP (either naturally or recombinantly) are cultured under conditions which will promote BiP/protein complexes. (Hela cells are one example of such cells.) Cells are washed twice in PBS (13.7 mM NaCl, 2.7 mM KCl, 80.9 mM Na₂HPO₄, pH 7.4) and then lysed by the addition of lysis buffer (50 mM HEPES, pH 7.5, 1% Triton X-100, 200 mM NaCl, 1.5 mM MgCl₂, 1 mM PMSF, 5 *µ*g/ml each aprotinin and leupeptin). Cell lysates are run through pre-clearing columns linked in line to an immunoaffinity column containing anti-BiP antibody. The column is washed and the BiP ligands released by the removal of Ca²⁺ or the addition of excess ATP. These ligands are first separated by size exclusion chromatography (SEC) to separate the smaller peptides from the larger proteins known to interact with BiP. Peptides isolated from BiP are further separated by reversed-phase chromatography (RPC) immediately after SEC fractionation and prior to mass analysis and sequence identification. Proteins isolated by SEC are further purified by ion exchange. Proteins isolated in this manner are digested using trypsin, and the subsequent cleavage products separated by RPC and identified by mass mapping or sequence identification using mass spectrometry.

### Example 8: Generation of Calnexin-Specific Ligand Profiles

The following example describes the generation of calnexin-specific protein profiles. As calnexin is an ER-specific transmembrane protein that selectively associates in a transient fashion with newly synthesized monomeric glycoproteins, in particular secretory proteins (Ou *et al*., 1993, *Nature* 364:771), it is a powerful multi-ligand receptor for the selective profiling of glycoproteins in any given cell that expresses calnexin, either naturally or recombinantly.

Calnexin expressing cells of interest (e.g., HepG2 cells (human hepatocellular carcinoma, ATCC No. HB-8065) (US Patent No. 4,393,133)) are grown in DMEM (GIBCO BRL, Gaithersburg, MD) supplemented with 10% FCS at 37°C and 5% CO₂. When confluent, cells are exposed to azetidine-2-carboxylic acid (Azc) for 60 minutes to enhance isolation of the calnexin-associated proteins (Ou *et al*., 1993, *supra).* Following this incubation period, cells are washed twice in PBS (13.7 mM NaCl, 2.7 mM KCl, 80.9 mM Na₂HPO₄, pH 7.4) and then lysed by the addition of lysis buffer (50 mM HEPES, pH 7.5, 2% sodium deoxycholate, 200 mM NaCl, 1.5 mM MgCl₂,

1 mM PMSF, 5 *µ*g/ml each aprotinin and leupeptin). To enhance isolation of calnexin binding ligands, one can substitute 1% digitonin or 0.5% Triton X-100 for the sodium deoxy cholate (Hochstenbach *et al*., 1992, *Proc. Natl. Acad. Sci. USA* 89:4734). Cell lysates are run through pre-clearing columns linked in line to an immunoaffinity column containing anti-calnexin antibody. The column is washed and the calnexin ligands released by the removal of Ca²⁺ (with a chelator such as EGTA) or the addition of excess ATP. These ligands are first fractionated by size exclusion chromatography (SEC) to separate the smaller peptides from the larger proteins known to interact with calnexin. Peptides isolated from calnexin are further separated by reversed-phase chromatography (RPC) immediately after SEC fractionation and prior to mass analysis and sequence identification. Proteins isolated by SEC are, optionally, further purified by ion exchange. Proteins isolated in this manner are then digested using trypsin, with the subsequent cleavage products separated by RPC and identified by mass mapping or sequence identification using mass spectrometry.

Other chaperones, chaperonins and hsps with properties similar to that of BiP and calnexin can be isolated as described above. For example, p72/74, another member of the heat shock family of proteins (VanBusKirk *et al.,* 1989, *J. Exp. Med.* 170:1799) is found in the lumen of the ER (VanBusKirk et *al.,* 1991, *J*. *Immuno.* 146:500), binds to peptides and ATP, and releases peptide upon ATP binding (Lakey *et al*., 1987, *Proc. Natl. Acad. sci. USA* 84:1659; DeNagel *et al.,* 1992, *Immun. Today* 13:86).

### Example 9: Generation of GP96/GRP94 EPT Profiles

The following example describes the generation of GP96/GRP94 EPT profiles. As GP96/GRP94 is a member of the HSP90 family of stress proteins present in the endoplasmic reticulum, it is a powerful multi-ligand receptor for the selective profiling of EPT libraries.

GP96/GRP94 is purified from liver cells as described (Blachere *et al*., 1997, *J. Exp. Med.* 186:1315; Nieland *et al*., 1996, *Proc. Natl.* Acad. *Sci. USA* 93:6135). Briefly, liver cells are homogenized in 40 ml hypotonic buffer (30 mM NaHCO₃, 0.1 mM phenylmethylsulfonyl fluoride, pH 7.1), and a 100,000 x g supernatant is obtained. The supernatant is fractionated by 50-70% ammonium sulfate precipitation, and that fraction is applied to a concanavalin A-affinity column. Protein elution is accomplished with 10% α-methylmannoside. The eluate is next loaded onto an anion exchange column equilibrated with 0.3 M NaCl; GP96/GRP94 is eluted with 0.7 M NaCl. EPT ligands can be extracted from the purified GP96/GRP94 multi-ligand binding receptors using acid elution as described previously for MHC-associated EPT profiles. Once the EPTs are extracted, generation of the EPT profile is identical to the procedures described for MHC-associated EPT profiles.

### Example 10: Generation of hsp 70 EPT Profiles

The following example describes the generation of hsp 70 EPT profiles. hsp 70 is a member of the HSP family of stress proteins that is present in various cellular compartments. It is a powerful multi-ligand receptor for the selective profiling of EPT libraries of cells in which hsp 70 is expressed (e.g., liver cells).

hsp 70 is purified from liver cells as described (Peng, 1997, *J*. *Immunol. Methods* 204:13). Briefly, liver cells are homogenized in 40 ml hypotonic buffer (30 mM NaHCO₃, 0.1 mM phenylmethylsulfonyl fluoride, pH 7.1), and a 100,000 x g supernatant is obtained. The sample buffer is changed to 20 mM Tris-acetate, 20 mM NaCl, 15 mM β-mercaptoethanol, 3 mM MgCl₂, 0.5 mM phenylmethylsulfonyl fluoride, pH 7.5, using a PD-10 column (Sephadex G-25). The sample is applied directly to an ADP-affinity column which has been equilibrated with the same buffer described above. hsp 70 elution is accomplished using 3 mM ADP at room temperature. The hsp 70 is next purified using a strong anion exchange column (Mono Q) and eluted with a 20-600 mM NaCl gradient. EPT ligands can be extracted from the hsp 70 multi-ligand binding receptor using acid elution as described previously for MHC-associated EPT profiles. Once the EPTs are extracted, generation of the EPT profile is identical to the procedures described for MHC-associated EPT profiles.

## Claims

1. A method of characterizing a given cell type, which cell type comprises a selected type of multi-ligand binding receptor, by generating a reproducible ligand profile for the cell type, the method comprising:
(a) providing a first sample of the given cell type, wherein the first sample comprises a first plurality of polypeptide ligands bound to the selected type of multi-ligand binding receptor;
(b) isolating the selected type of multi-ligand binding receptor from the first sample;
(c) separating the first plurality of ligands from the selected type of multi-ligand binding receptor;
(d) fractionating the first plurality of ligands;
(e) generating a first profile distinguishing among the first plurality of ligands on the basis of at least one chemical or physical attribute;
(f) providing a second sample of the given cell type, the second sample being essentially identical to the first sample, wherein the second sample comprises a second plurality of polypeptide ligands bound to the selected type of multi-ligand binding receptor;
(g) isolating the selected type of multi-ligand binding receptor from the second sample;
(h) separating the second plurality of ligands from the selected type of multi-ligand binding receptor;
(i) fractionating the second plurality of ligands;
(j) generating a second profile distinguishing among the second plurality of ligands on the basis of the at least one chemical or physical attribute; and
(k) confirming that the first profile and the second profile are essentially identical, and together represent a reproducible ligand profile for the given cell type.

2. The method of claim 1, wherein a second chemical or physical attribute of each ligand is determined subsequent to the fractionation steps, and is represented in the profiles.

3. The method of claim 2, wherein a third chemical or physical attribute of each ligand is determined subsequent to the fractionation steps, and is represented in the profiles.

4. The method of claim 1, wherein the isolating and separating steps are accomplished using appropriate columns arranged in an in-line system.

5. A method of characterizing a given cell type, which cell type comprises a selected type of multi-ligand binding receptor, by generating a ligand profile for the cell type, comprising:
(a) providing a sample of lysate of the given type of cell, wherein the sample comprises a first plurality of polypeptide ligands bound to a first type of multi-ligand binding receptor and a second plurality of polypeptide ligands bound to a second type of multi-ligand binding receptor;
(b) isolating the first and second types of multi-ligand binding receptors from the sample;
(c) separating the first plurality of ligands from the first type of multi-ligand binding receptor and the second plurality of ligands from the second type of multi-ligand binding receptor;
(d) fractionating the first plurality of ligands and the second plurality of ligands; and
(e) generating a first profile distinguishing among the first plurality of ligands on the basis of at least one chemical or physical attribute and a second profile distinguishing among the second plurality of ligands on the basis of the same at least one chemical or physical attribute.

6. A method of generating a subtraction profile of polypeptide ligands, comprising:
(a) producing a first ligand profile by a method comprising:
(i) providing a first sample comprising a first cell of interest, wherein the first cell of interest comprises a given type of multi-ligand binding receptor bound to a first set of polypeptide ligands;
(ii) isolating the given type of multi-ligand binding receptor and the first set of ligands from the first sample;
(iii) separating the first set of ligands from the given type of multi-ligand binding receptor;
(iv) generating a first profile distinguishing among the first set of ligands on the basis of at least one chemical or physical attribute;
(b) producing a second profile of ligands by a method comprising:
(i) providing a second sample comprising a second cell of interest, wherein the second cell of interest comprises the given type of multi-ligand binding receptor, bound to a second set of polypeptide ligands;
(ii) isolating the given type of multi-ligand binding receptor and the second set of ligands from the second sample;
(iii) separating the second set of ligands from the given type of multi-ligand binding receptor;
(iv) generating a second profile distinguishing among the second set of ligands on the basis of the same at least one chemical or physical attribute;
(c) comparing the first profile and the second profile to identify differentially expressed ligands, thereby forming a subtraction profile of ligands.

7. A method of comparing a first cell sample to a reference cell sample, comprising:
(a) producing a first ligand profile by a method comprising:
(i) providing a first cell sample comprising a given type of multi-ligand binding receptor bound to a first set of polypeptide ligands;
(ii) isolating the given type of multi-ligand binding receptor and the first set of ligands from the first cell sample;
(iii) separating the first set of ligands from the given type of multi-ligand binding receptor;
(iv) generating a first ligand profile distinguishing among the first set of ligands on the basis of at least one chemical or physical attribute;
(b) providing a reference ligand profile representing a second set of polypeptide ligands extracted from the given type of multi-ligand binding receptor of a reference cell sample, wherein the reference ligand profile distinguishes among the second set of polypeptide ligands on the basis of the at least one chemical or physical attribute; and
(c) comparing the first ligand profile to the reference ligand profile, in order to identify differences or similarities between the first cell sample and the reference cell sample.

8. The method of claim 7, wherein the reference cell sample consists essentially of healthy cells of an animal and the first cell sample comprises cells suspected of being diseased.

9. The method of claim 7, wherein the first cell sample comprises cells cultured in the presence of a test compound, and the reference cell sample does not.

10. The method of claim 7, wherein the reference cell sample comprises cells cultured in the presence of a test compound, and the first cell sample does not.

11. A method of detecting a difference between the set of proteins expressed in a first cell and the set of proteins expressed in a second cell, comprising the method of claim 6, wherein any difference identified between the first profile and the second profile is an indication of the difference between the set of proteins expressed in the first cell and the set of proteins expressed in the second cell.

12. The method of claim 11, comprising the further step of
(d) generating a differential profile which sets forth at least some of the differences between the set of proteins expressed in the first cell and the set of proteins expressed in the second cell.

13. The method of claim 11, comprising the further steps of selecting a ligand which is represented in one profile but not in the other, and identifying the amino acid sequence of the ligand.

14. The method of any of claims 1-13, wherein the multi-ligand binding receptor is an MHC class I or MHC class II receptor.

15. The method of claim 14, wherein at least 500 polypeptide ligands are represented in the ligand profile.

16. The method of any of claims 1-13, wherein the multi-ligand binding receptor is not an MHC class I or MHC class II receptor.

17. The method of any of claims 1-13, wherein the multi-ligand binding receptor is a chaperone, a calnexin, a calreticutin, a mannosidase, a N-glycanase, a BIP, a grp94, a grp96, an E2 ubiquitin carrier protein, an E3 ubiquitin ligase, an unfoldase, a proteasome, a trafficking protein, or a retention protein.

18. The method of any of claims 1-13, wherein the multi-ligand binding receptor is a chaperone selected from the group consisting of a chaperonin, hsp60, hsp65, hsp70, hsp90, hsp25, and hsp100.

19. The method of any of claims 1-13, wherein the at least one chemical or physical attribute comprises hydrophobicity or charge.

20. The method of any of claims 1-13, wherein the at least one chemical or physical attribute comprises mass-to-charge ratio.

21. The method of any of claims 1-13, wherein the at least one chemical or physical attribute comprises amino acid sequence.

## Patentansprüche

1. Verfahren zur Charakterisierung einer bestimmten Zellart, wobei die Zellart einen ausgewählten Multi-Ligandenbindungsrezeptor-Typ umfasst, durch das Erstellen eines reproduzierbaren Ligandenprofils der Zellart, wobei das Verfahren umfasst:
(a) Bereitstellen einer ersten Probe der bestimmten Zellart, wobei die erste Probe eine erste Vielzahl von Polypeptidliganden gebunden an den ausgewählten Multi-Ligandenbindungsrezeptor-Typ umfasst;
(b) Isolieren des ausgewählten Multi-Ligandenbindungsrezeptor-Typs von der ersten Probe;
(c) Abtrennen der ersten Vielzahl von Liganden von dem ausgewählten Multi-Ligandenbindungsrezeptor-Typ;
(d) Fraktionieren der ersten Vielzahl von Liganden;
(e) Erstellen eines ersten Profils, das unter der ersten Vielzahl von Liganden basierend auf mindestens einer chemischen oder physikalischen Eigenschaft unterscheidet;
(f) Bereitstellen einer zweiten Probe der bestimmten Zellart, wobei die zweite Probe im Wesentlichen identisch ist mit der ersten Probe, wobei die zweite Probe eine zweite Vielzahl von Polypeptidliganden gebunden an den ausgewählten Multi-Ligandenbindungsrezeptor-Typ umfasst;
(g) Isolieren des ausgewählten Multi-Ligandenbindungsrezeptor-Typs von der zweiten Probe;
(h) Abtrennen der zweiten Vielzahl von Liganden von dem ausgewählten Multi-Ligandenbindungsrezeptor-Typ;
(i) Fraktionieren der zweiten Vielzahl von Liganden;
(j) Erstellen eines zweiten Profils, das unter der zweiten Vielzahl von Liganden basierend auf mindestens einer chemischen oder physikalischen Eigenschaft unterscheidet; und
(k) Bestätigen, dass das erste Profil und das zweite Profil im Wesentlichen identisch sind und gemeinsam ein reproduzierbares Ligandenprofil für die bestimmte Zellart darstellen.

2. Verfahren nach Anspruch 1, wobei anschließend an die Fraktionierungsschritte eine zweite chemische oder physikalische Eigenschaft von jedem Liganden bestimmt und in den Profilen dargestellt wird.

3. Verfahren nach Anspruch 2, wobei anschließend an die Fraktionierungsschritte eine dritte chemische oder physikalische Eigenschaft von jedem Liganden bestimmt und in den Profilen dargestellt wird.

4. Verfahren nach Anspruch 1, wobei die Isolierungs- und Abtrennungsschritte unter Verwendung geeigneter Säulen erfolgen, die in Reihe angeordnet sind.

5. Verfahren zur Charakterisierung einer bestimmten Zellart, wobei die Zellart einen ausgewählten Multi-Ligandenbindungsrezeptor-Typ umfasst, durch das Erstellen eines Ligandenprofils der Zellart, umfassend:
(a) Bereitstellen einer Probe eines Lysates der bestimmten Zellart, wobei die Probe eine erste Vielzahl von an einen ersten Multi-Ligandenbinungsrezeptor-Typ gebundenen Polypeptidliganden und eine zweite Vielzahl von an einen zweiten Multi-Ligandenbindungsrezeptor-Typ gebundenen Polypeptidliganden umfasst;
(b) isolieren der ersten und zweiten Multi-Ligandenbindungsrezeptor-Typen von der Probe;
(c) Abtrennen der ersten Vielzahl von Liganden von dem ersten Multi-Ligandenbindungsrezeptor-Typ und der zweiten Vielzahl von Liganden von dem zweiten Multi-Ligandenbindungsrezeptor-Typ;
(d) Fraktionieren der ersten Vielzahl von Liganden und der zweiten Vielzahl von Liganden; und
(e) Erstellen eines ersten Profils, das unter der ersten Vielzahl von Liganden basierend auf mindestens einer chemischen oder physikalischen Eigenschaft unterscheidet, und eines zweiten Profils, das unter der zweiten Vielzahl von Liganden basierend auf der gleichen mindestens einen chemischen oder physikalischen Eigenschaft unterscheidet.

6. Verfahren zum Erstellen eines Subtraktionsprofils von Polypeptidliganden, umfassend:
(a) Erzeugen eines ersten Ligandenprofils mittels eines Verfahrens umfassend:
(i) Bereitstellen einer ersten Probe, umfassend eine Zelle von Interesse, wobei die erste Zelle von Interesse einen bestimmten an einen ersten Satz von Polypeptidliganden gebundenen Multi-Ligandenbindungsrezeptor-Typ umfasst;
(ii) Isolieren des bestimmten Multi-Ligandenbindungsrezeptor-Typs und des ersten Satzes von Liganden aus der ersten Probe;
(iii) Abtrennen des ersten Satzes von Liganden von dem bestimmten Multi-Ligandenbindungsrezeptor-Typ;
(iv) Erstellen eines ersten Profils, das unter dem ersten Satz von Liganden basierend auf mindestens einer chemischen oder physikalischen Eigenschaft unterscheidet;
(b) Erzeugen eines zweiten Profils von Liganden mittels eines Verfahrens, umfassend:
(i) Bereitstellen einer zweiten Probe, umfassend eine zweite Zelle von Interesse, wobei die zweite Zelle von Interesse den bestimmten an einen zweiten Satz von Polypeptidliganden gebundenen Multi-Ligandenbindungsrezeptor-Typ umfasst;
(ii) Isolieren des bestimmten Multi-Ligandenbindungsrezeptor-Typs und des zweiten Satzes von Liganden aus der zweiten Probe;
(iii) Abtrennen des zweiten Satzes von Liganden von dem bestimmten Multi-Ligandenbindungsrezeptor-Typ;
(iv) Erstellen eines zweiten Profils, das unter dem zweiten Satz von Liganden basierend auf der gleichen mindestens einen chemischen oder physikalischen Eigenschaft unterscheidet;
(c) Vergleichen des ersten Profils mit dem zweiten Profil zur Identifizierung von unterschiedlich exprimierten Liganden, wodurch ein Subtraktionsprofil von Liganden erstellt wird.

7. Verfahren zum Vergleichen einer ersten Zellprobe mit einer Referenzzellprobe, umfassend:
(a) Herstellen eines ersten Ligandenprofils mittels eines Verfahrens umfassend:
(i) Bereitstellen einer ersten Zellprobe, umfassend einen bestimmten Multi-Ligandenbindungsrezeptor-Typ gebunden an einen ersten Satz von Polypeptidliganden;
(ii) Isolieren des bestimmten Multi-Ligandenbindungsrezeptor-Typs und des ersten Satzes von Liganden von der ersten Zellprobe;
(iii) Abtrennen des ersten Satzes von Liganden von dem bestimmten Multi-Ligandenbindungsrezeptor-Typ;
(iv) Erstellen eines ersten Ligandenprofils, das unter dem ersten Satz von Liganden basierend auf mindestens einer chemischen oder physikalischen Eigenschaft unterscheidet;
(b) Bereitstellen eines Referenzligandenprofils, das einen zweiten Satz von Polypeptidliganden repräsentiert, die von dem bestimmten Multi-Ligandenbindungsrezeptor-Typ einer Referenzzellprobe isoliert wurden, wobei das Referenzligandenprofil unter dem zweiten Satz von Polypeptidliganden basierend auf mindestens einer chemischen oder physikalischen Eigenschaft unterscheidet; und
(c) Vergleichen eines ersten Ligandenprofils mit dem Referenzligandenprofil zur Identifizierung von Unterschieden oder Ähnlichkeiten zwischen der ersten Zellprobe und der Referenzzellprobe.

8. Verfahren nach Anspruch 7, wobei die Referenzzellprobe im Wesentlichen aus gesunden tierischen Zellen besteht und die erste Zellprobe Zellen umfasst, bei denen ein Verdacht auf eine Erkrankung besteht.

9. Verfahren nach Anspruch 7, wobei die erste Zellprobe in Anwesenheit einer Testverbindung gezüchtete Zellen umfasst, und die Referenzzellprobe nicht.

10. Verfahren nach Anspruch 7, wobei die Referenzzellprobe in Anwesenheit einer Testverbindung gezüchtete Zellen umfasst, und die erste Zellprobe nicht.

11. Verfahren zur Erfassung eines Unterschieds zwischen dem Satz von Proteinen, die in einer ersten Zelle exprimiert werden, und dem Satz von Proteinen, die in einer zweiten Zelle exprimiert werden, umfassend das Verfahren nach Anspruch 6 wobei jeglicher Unterschied, der zwischen dem ersten Profil und dem zweiten Profil festgestellt wird, einen Hinweis auf den Unterschied zwischen dem Satz von Proteinen, die in der ersten Zelle exprimiert werden, und dem Satz von Proteinen, die in der zweiten Zelle exprimiert werden, darstellt.

12. Verfahren nach Anspruch 11, umfassend den weiteren Schritt des
(d) Erstellens eines Differenzialprofils, das mindestens einige der Unterschiede zwischen dem Satz von Proteinen, die in der ersten Zelle exprimiert werden, und dem Satz von Proteinen, die in der zweiten Zelle exprimiert werden, darlegt.

13. Verfahren nach Anspruch 11, umfassend die weiteren Schritte der Selektion eines Liganden, der in einem der Profile jedoch nicht in dem anderen Profil vertreten ist, und der Identifizierung der Aminosäuresequenz des Liganden.

14. Verfahren nach einem der Ansprüche 1 bis 13, wobei der Multi-Ligandenbindungsrezeptor ein MHC-Klasse-I- oder ein MHC-Klasse-II-Rezeptor ist.

15. Verfahren nach Anspruch 14, wobei mindestens 500 Polypeptidiganden in dem Ligandenprofil dargestellt werden.

16. Verfahren nach einem der Ansprüche 1 bis 13, wobei der Multi-Ligandenbindungsrezeptor kein MHC-Klasse-I- oder MHC-Klasse-II Rezeptor ist.

17. Verfahren nach einem der Ansprüche 1 bis 13, wobei der Multi-Ligandenbindungsrezeptor ein Chaperon, ein Calnexin, ein Calreticutin, eine Mannosidase, eine N-Glycanase, ein BIP, ein grp94, ein grp96, ein E2-Ubiquitin-Trägerprotein, eine E3-Ubiquitin-Ligase, eine Unfoldase, ein Proteasom, ein Trafficking-Protein oder ein Retentionsprotein ist.

18. Verfahren nach einem der Ansprüche 1 bis 13, wobei der Multi-Ligandenbindungsrezeptor ein Chaperon ist, ausgewählt aus der Gruppe bestehend aus einem Chaperonin, hsp60, hsp65, hsp70, hsp90, hsp25 und hsp100.

19. Verfahren nach einem der Ansprüche 1 bis 13, wobei mindestens eine chemische oder physikalische Eigenschaft Hydrophobizität oder Ladung umfasst.

20. Verfahren nach einem der Ansprüche 1 bis 13, wobei mindestens eine chemische oder physikalische Eigenschaft das Masse-zu-Ladung-Verhältnis umfasst.

21. Verfahren nach einem der Ansprüche 1 bis 13, wobei mindestens eine chemische oder physikalische Eigenschaft eine Aminosäuresequenz umfasst.

## Revendications

1. Procédé de caractérisation d'un type cellulaire déterminé, type cellulaire qui comporte un type choisi de récepteur se liant à de multiples ligands, par la production d'un profil de ligands reproductible pour le type cellulaire, le procédé consistant à :
(a) fournir un premier échantillon du type cellulaire déterminé, où le premier échantillon comprend une première pluralité de ligands polypeptidiques liés au type choisi de récepteur se liant à de multiples ligands ;
(b) isoler le type choisi de récepteur se liant à de multiples ligands du premier échantillon ;
(c) séparer la première pluralité de ligands du type choisi de récepteur se liant à de multiples ligands ;
(d) fractionner la première pluralité de ligands ;
(e) produire un premier profil faisant la distinction dans la première pluralité de ligands d'après au moins une propriété chimique ou physique ;
(f) fournir un second échantillon du type cellulaire déterminé, le second échantillon étant essentiellement identique au premier échantillon, où le second échantillon comprend une seconde pluralité de ligands polypeptidiques liés au type choisi de récepteur se liant à de multiples ligands ;
(g) isoler le type choisi de récepteur se liant à de multiples ligands du second échantillon ;
(h) séparer la seconde pluralité de ligands du type choisi de récepteur se liant à de multiples ligands ;
(i) fractionner la seconde pluralité de ligands ;
(j) produire un second profil faisant la distinction dans la seconde pluralité de ligands d'après au moins une propriété chimique ou physique ; et
(k) confirmer que le premier profil et le second profil sont essentiellement identiques, et qu'ils représentent ensemble un profil de ligands reproductible pour le type cellulaire déterminé.

2. Procédé de la revendication 1, dans lequel une seconde propriété chimique ou physique de chaque ligand est établie à la suite des étapes de fractionnement, et est représentée dans les profils.

3. Procédé de la revendication 2, dans lequel une troisième propriété chimique ou physique de chaque ligand est établie à la suite des étapes de fractionnement, et est représentée dans les profils.

4. Procédé de la revendication 1, dans lequel les étapes d'isolement et de séparation sont accomplies en utilisant des colonnes convenables et montées dans un système en série.

5. Procédé de caractérisation d'un type cellulaire déterminé, type cellulaire qui comporte un type choisi de récepteur se liant à de multiples ligands, par la production d'un profil de ligands reproductible pour le type cellulaire, consistant à :
(a) fournir un échantillon de lysat du type de cellule déterminé, où l'échantillon comprend une première pluralité de ligands polypeptidiques liés à un premier type de récepteur se liant à de multiples ligands et une seconde pluralité de ligands polypeptidiques liés à un second type de récepteur se liant à de multiples ligands ;
(b) isoler le premier et le second type de récepteur se liant à de multiples ligands de l'échantillon ;
(c) séparer la première pluralité de ligands du premier type de récepteur se liant à de multiples ligands et la seconde pluralité de ligands du second type de récepteur se liant à de multiples ligands ;
(d) fractionner la première pluralité de ligands et la seconde pluralité de ligands ; et
(e) produire un premier profil faisant la distinction dans la première pluralité de ligands d'après au moins une propriété chimique ou physique et un second profil faisant la distinction dans la seconde pluralité de ligands d'après la même au moins une propriété chimique ou physique.

6. Procédé de production d'un profil de ligands polypeptidiques par soustraction, consistant à :
(a) produire un premier profil de ligands par un procédé consistant à:
(i) fournir un premier échantillon comprenant une première cellule d'intérêt, où la première cellule d'intérêt comporte un type déterminé de récepteur se liant à de multiples ligands lié à une première série de ligands polypeptidiques ;
(ii) isoler le type déterminé de récepteur se liant à de multiples ligands et la première série de ligands du premier échantillon ;
(iii) séparer la première série de ligands du type déterminé de récepteur se liant à de multiples ligands ;
(iv) produire un premier profil faisant la distinction dans la première série de ligands d'après au moins une propriété chimique ou physique ;
(b) produire un second profil de ligands par un procédé consistant à :
(i) fournir un second échantillon comprenant une seconde cellule d'intérêt, où la seconde cellule d'intérêt comporte le type déterminé de récepteur se liant à de multiples ligands, lié à une seconde série de ligands polypeptidiques ;
(ii) isoler le type déterminé de récepteur se liant à de multiples ligands et la seconde série de ligands du second échantillon ;
(iii) séparer la seconde série de ligands du type déterminé de récepteur se liant à de multiples ligands ;
(iv) produire un second profil faisant la distinction dans la seconde série de ligands d'après la même au moins une propriété chimique ou physique ;
(c) comparer le premier profil et le second profil afin d'identifier des ligands exprimés de manière différente, et créer de ce fait un profil de ligands par soustraction.

7. Procédé de comparaison d'un premier échantillon cellulaire à un échantillon cellulaire de référence, consistant à :
(a) produire un premier profil de ligands par un procédé consistant à:
(i) fournir un premier échantillon cellulaire comprenant un type déterminé de récepteur se liant à de multiples ligands lié à une première série de ligands polypeptidiques ;
(ii) isoler le type déterminé de récepteur se liant à de multiples ligands et la première série de ligands du premier échantillon cellulaire ;
(iii) séparer la première série de ligands du type déterminé de récepteur se liant à de multiples ligands ;
(iv) produire un premier profil de ligands faisant la distinction dans la première série de ligands d'après au moins une propriété chimique ou physique ;
(b) fournir un profil de ligands de référence représentant une seconde série de ligands polypeptidiques extraite du type déterminé de récepteur se liant à de multiples ligands d'un échantillon cellulaire de référence, où le profil de ligands de référence fait la distinction dans la seconde série de ligands polypeptidiques d'après la au moins une propriété chimique ou physique ; et
(c) comparer le premier profil de ligands avec le profil de ligands de référence, afin de d'identifier des différences ou des similitudes entre le premier échantillon cellulaire et l'échantillon cellulaire de référence.

8. Procédé de la revendication 7, dans lequel l'échantillon cellulaire de référence se compose essentiellement de cellules saines d'un animal et le premier échantillon cellulaire se compose de cellules soupçonnées d'être malades.

9. Procédé de la revendication 7, dans lequel le premier échantillon cellulaire se compose de cellules cultivées en présence d'un composé test, et l'échantillon cellulaire de référence pas.

10. Procédé de la revendication 7, dans lequel l'échantillon cellulaire de référence se compose de cellules cultivées en présence d'un composé test, et le premier échantillon cellulaire pas.

11. Procédé de détection d'une différence entre la série de protéines exprimées dans une première cellule et la série de protéines exprimées dans une seconde cellule, consistant en le procédé de la revendication 6, dans lequel toute différence identifiée entre le premier profil et le second profil constitue une indication de la différence entre la série de protéines exprimées dans la première cellule et la série de protéines exprimées dans la seconde cellule.

12. Procédé de la revendication 11, comprenant l'étape supplémentaire consistant à
(d) produire un profil différentiel qui expose au moins quelques unes des différences entre la série de protéines exprimées dans la première cellule et la série de protéines exprimées dans la seconde cellule.

13. Procédé de la revendication 11, comprenant les étapes supplémentaires consistant à choisir un ligand qui est représenté dans un profil mais pas dans l'autre, et à identifier la séquence d'acides aminés du ligand.

14. Procédé de l'une quelconque des revendications 1 à 13, dans lequel le récepteur se liant à de multiples ligands est un récepteur du CMH de classe 1 ou du CMH de classe II.

15. Procédé de la revendication 14, dans lequel au moins 500 ligands polypeptidiques sont représentés dans le profil de ligands.

16. Procédé de l'une quelconque des revendications 1 à 13, dans lequel le récepteur se liant à de multiples ligands n'est pas un récepteur du CMH de classe I ou du CMH de classe II.

17. Procédé de l'une quelconque des revendications 1 à 13, dans lequel le récepteur se liant à de multiples ligands est une protéine chaperon, une calnexine, une calréticuline, une mannosidase, une N-glycanase, une BIP, une grp94, une grp96, une protéine porteuse d'ubiquitine E2, une ubiquitine ligase E3, une « unfoldase », un protéasome, une protéine de régulation du trafic, ou une protéine de rétention.

18. Procédé de l'une quelconque des revendications 1 à 13, dans lequel le récepteur se liant à de multiples ligands est une protéine chaperon choisie dans le groupe formé par une chaperonine, hsp60, hsp65, hsp70, hsp90, hsp25, et hsp100.

19. Procédé de l'une quelconque des revendications 1 à 13, dans lequel la au moins une propriété chimique ou physique consiste en l'hydrophobicité ou la charge.

20. Procédé de l'une quelconque des revendications 1 à 13, dans lequel la au moins une propriété chimique ou physique consiste en le rapport entre la masse et la charge.

21. Procédé de l'une quelconque des revendications 1 à 13, dans lequel la au moins une propriété chimique ou physique consiste en la séquence en acides aminés.
